# EUROPEAN PATENT SPECIFICATION

(11) **EP 4 622 990 B1**
(45) Date of publication and mention of the grant of the patent: **28.10.2026**
(21) Application number: 23809578.0
(22) Date of filing: 21.11.2023
(51) Int. Cl.: C07K 1/22

(54) **METHOD FOR PURIFICATION OF A TARGET PRODUCT USING AFFINITY PURIFICATION TECHNOLOGY**
VERFAHREN ZUR REINIGUNG EINES ZIELPRODUKTS MITTELS AFFINITÄTSREINIGUNGSTECHNOLOGIE
PROCÉDÉ DE PURIFICATION D'UN PRODUIT CIBLE À L'AIDE D'UNE TECHNOLOGIE DE PURIFICATION PAR AFFINITÉ

(30) Priority: 21.11.2022 EP 22208670
(43) Date of publication of application: 01.10.2025
(73) Proprietor: CHRETO ApS, 2970 Hørsholm (DK)
(72) Inventor: KYHSE-ANDERSEN, Jan, 3500 Værløse (DK); WINTHER, Lars, 2765 Smørum (DK)
(74) Representative: Inspicos P/S
(86) International application number: PCT/EP2023/082500
(87) International publication number: WO 2024/110444

(56) References cited:
- EP-B1- 2 427 482
- WO-A1-2016/049761
- WO-A2-2009/062942
- WO-A2-2009/078018
- WO-A2-2010/128033
- FORDE GARETH M ED - DUCRY ET AL: "Plasmid DNA Purification Via the Use of a Dual Affinity Protein", ANTIBODY-DRUG CONJUGATES; [METHODS IN MOLECULAR BIOLOGY; ISSN 1064-3745], HUMANA PRESS, US, vol. 421, 1 January 2008 (2008-01-01), pages 275 - 283, XP008130755, ISBN: 978-1-62703-541-5, DOI: 10.1007/978-1-59745-582-4_18
- MONDAL K ET AL: "The affinity concept in bioseparation: Evolving paradigms and expanding range of applications", BIOMOLECULAR ENGINEERING, ELSEVIER, NEW YORK, NY, US, vol. 23, no. 2-3, 1 June 2006 (2006-06-01), pages 59 - 76, XP025182531, ISSN: 1389-0344, [retrieved on 20060601], DOI: 10.1016/J.BIOENG.2006.01.004

## Description

### FIELD OF THE INVENTION

The present invention relates to a general affinity purification technology using a dual affinity polypeptide. In particular, the invention relates to a fast and efficient method for removing a dual affinity protein from a target molecule in solution.

### BACKGROUND OF THE INVENTION

The currently most common method of affinity purification of biomolecules, such as proteins, is affinity column chromatography, where target biomolecules are bound to an affinity ligand immobilised on column resin or solid matrix, washed and subsequently recovered by elution from the column resin.

The affinity solid supports, resin beads, matrices or solid phases are functionalized with specific target binding molecules or ligands and are typically packed in columns.

Resin beads used in affinity purification are produced from polymers using chemical crosslinking reactions. Typically, these beads have a size in the order of 90-100 µm and contain pores in the order of about 30 nm. Both the outer and inner surfaces of the beads are available for attaching the affinity ligands.

The outer surface area of these beads is about 1/100 of the inner surface area. This means that only a small part of the bead surface is accessible for ligand attachment on the outside, while the majority of ligands are fixed within the resin bead.

Consequently, the product molecules can only interact with the attached affinity ligands by going through a diffusion-based mass transport to the inner surface of the beads. This diffusion process takes time, and it is a consistent feature across all resin chromatography processes, whether in laboratory, pilot, or industrial scales. The current invention aims to eliminate or at least diminish these technical limitations.

The traditional affinity purification involves the following general steps:
A. Binding the target by incubating a crude sample (e.g., cell lysate or plasma) with an affinity solid support to allow the target molecule in the sample to bind to an immobilized ligand;
B. Washing away impurities (non-bound sample components) from the solid support using appropriate buffers that maintain the binding interaction between target and affinity ligand;
C. Eluting and recover the target molecule from the immobilized ligand by altering the buffer conditions so the binding interaction between target and ligand no longer occurs; followed by
D. Regeneration or cleaning-in-place (CIP) - if the affinity ligand solid support is for reuse.

EP 2220107 B1 discloses a process for purification of a target biomolecule, comprising the steps: (a) contacting (i) a target biomolecule, (ii) a dual affinity polypeptide, and (iii) a solid substrate comprising a catching ligand; and (b) recovering the target biomolecule by elution, wherein the target biomolecule and the dual affinity polypeptide are contacted in solution before the mixture is contacting the solid substrate.

EP 2427482 B1 discloses a process for purification of a target molecule, comprising the steps: (a) contacting a target molecule, and a population of target binding polypeptides (TBP), in solution for a sufficient time to allow complex formation; and (b) isolating the target from the complex from (a) by subsequent purification steps, wherein (i) the target binding polypeptides have at least two binding functionalities; a first binding functionality towards the target and a second binding functionality towards a catching ligand comprised in a solid substrate; and (ii) the first binding functionality comprises at least two binding sites for the target, and the target comprises at least two binding sites for the TBP.

The prior art methods utilize resin column or solid matrix purification processes where the resins are to optimized with respect to particle size to control the surface area, the binding capacity and backpressure in columns. This is done also to optimize the overall purification efficiency and minimize the operational cost.

The most commonly used packed bed resin columns are prone to increased back pressure and uneven flow patterns when scaled up due to resin properties which may lead to collapse of the resin. The productivity with respect to amount of purified target product per hour, time for cleaning in place and overall operational costs is closely associated with the column size, format and resin properties. When scaling up purification processes it is therefore difficult to increase the purification capacity or reduce the size and footprint of column-based systems. Also, it is difficult to reduce the process water consumption, as cleaning in place (CIP) is necessary when reusing the expensive affinity column resins.

Purification of targets by affinity chromatography using packed columns is widely known. This is described in e.g. WO2023012321 A1.

Multiple variations of the traditional technology try to solve the previously mentioned drawbacks.

EP 2220107 B1 and EP 2427482 B1 describe purification with Protein A-Streptavidin fusion proteins catching the target on solid substrate, followed by elution and recovery of the target. These technologies use a solid resin matrix for capturing the fusion protein.

Purification procedures with Protein A attached to a soluble polymer is described in US2009232737 A1 and WO2016049761 A1. The chromatographic material is a soluble polymer, with chemically attached binding moieties (e.g., antigens) against the target to be purified. The polymer can be precipitated by change of temperature or pH. The target product is purified by first binding in solution with the polymer, precipitation of polymer, washing steps and elution of the target protein from the polymer.

Other affinity purification procedures of target proteins are described in US2008108053 A1, WO2020037100 A1, WO2012055854 A1, WO2018178991 A1, WO2021168270 A1, WO2006110292 A2, WO2009078018 A2, WO2008067591 A1, which all describe various affinity purification systems with a Protein A anchor fusion protein containing moieties, which can precipitate the entire target-protein A fusion protein complex. The precipitation is done by e.g., adding metal ions, changing temperature or pH. The precipitated complex is collected and washed and the target eluted from the complex and collected. All of the above-mentioned technologies are as conventional affinity purification procedures focused on capturing and purifying the target proteins by first binding to the target, washing steps, followed by an elution step and collection of the target protein.

Other publications of potential relevance include US 2012238039 A1, WO0031128 A1, WO2017167960 A1, US2013337528 A1, WO2022179970 A1, US2021024616 A1, WO2021007484 A1 and WO2013177115 A2. Still other publications of potential relevance include FORDE, G.M. (2008): "Plasmid DNA Purification Via the Use of a Dual Affinity Protein", in Zachariou, M. (eds) Affinity Chromatography, Methods in Molecular Biology, vol 421, pp. 275-284, Humana Press and MONDAL, K. et al. (2006): "The affinity concept in bioseparation: Evolving paradigms and expanding range of application", Biomolecular Engineering, Vol. 23, Issues 2-3, pp. 59-76, https://doi.org/10.1016/j.bioeng.2006.01.004.

There remains therefore a need for a procedure for purification of target molecules wherein reaction time and overall costs could be reduced significantly and productivity can be increased.

The inventors have realised that it would be preferable to apply a soluble matrix with covalently bound ligand to solve the time-consuming mass transport issues by diffusion and to establish a rapid and scalable purification procedure.

In a method for purification of a target biomolecule using a dual affinity polypeptide (DAP) the binding reaction (DAP binding to Biotin linker-matrix) was - to the inventor's surprise - so much faster in solution than when DAP was reacted with a biotin ligand immobilized on the inner surface of resin beads. The affinity binding reaction of a soluble ligand-matrix was in the order of seconds (90-150 sec) and much faster than ligands immobilised onto resin beads. In the latter case, the reaction time (residence time in a column) was much longer and in minutes (50-70 minutes).

The extended reaction time primarily results from the mass transport of molecules by diffusion to reach the inner surface of the beads. This delay is compounded by the established knowledge that chemical reactions occur about 1000 times faster in solution than for heterogeneous reactions on surfaces. (reference Nygren, H. and Stenberg, M. (1989) Immunochemistry at Interfaces. Immunology, 66, 321-327*)*

The fast affinity procedure, which is exemplified in this invention by removal of DAP from a solution of target biomolecules provides an example and background for novel process modalities like in line processing and continuous process designs for industrial applications.

There remains therefore a need for a procedure for purification of target molecules wherein reaction time and overall costs could be reduced significantly and productivity can be increased.

### OBJECT OF THE INVENTION

It is an object of embodiments of the invention to provide a method for purification of target molecules, which is scalable, results in reduced process time and thereby increased productivity and target product stability, and results in reduced process footprint and wastewater consumption.

### SUMMARY OF THE INVENTION

It has been found by the present inventors that by avoiding the use of solid matrices and resins columns and instead by utilising a soluble capture matrix an effective and industrially applicable scale-up method is provided, that results in shorter overall process time and a reduced residence time for the target biomolecule at e.g., low pH, which may lower the risk of denaturation or decomposition of the target biomolecules.

The inventors have realised that it would be preferable to apply a soluble matrix with covalently bound ligand to solve the time-consuming mass transport issues by diffusion and to establish a rapid and scalable purification procedure.

In a method for purification of a target biomolecule using a dual affinity polypeptide (DAP) the binding reaction (DAP binding to Biotin linker-matrix) was - to the inventor's surprise - so much faster in solution than when DAP was reacted with a biotin ligand immobilized on the inner surface of resin beads. The affinity binding reaction of a soluble ligand-matrix was in the order of seconds (90-150 sec) and much faster than ligands immobilised onto resin beads. In the latter case, the reaction time (residence time in a column) was much longer and in minutes (50-70 minutes).

The fast affinity procedure, which is exemplified in this invention by removal of DAP from a solution of target biomolecules provides an example and background for novel process modalities like in line processing and continuous process designs for industrial applications.

So, in a first aspect the present invention relates to a method for purification of a target biomolecule, comprising the steps: a. contacting i) a starting composition comprising the target biomolecule and ii) a dual affinity polypeptide (DAP), said DAP having a first binding functionality towards the target biomolecule and a second binding functionality towards a catching ligand, in solution, to allow the DAP to form 3D-complexes with the target biomolecule; b. washing the formed target biomolecule-DAP complexes to remove any impurities and to form pre-purified target biomolecule-DAP complexes; c. treating the pre-purified target biomolecule-DAP complexes at a pH below 5.0 to separate the pure target product biomolecule from the DAP by dissolving the 3D-complex and thereby providing a mixture of target product biomolecules and DAP; d. contacting the mixture of c. with a soluble matrix having the catching ligand covalently bound to it, to form separate pure target biomolecules and separate DAP- catching ligand-matrix products in solution; and e. recovering the pure target biomolecule.

### FIGURES

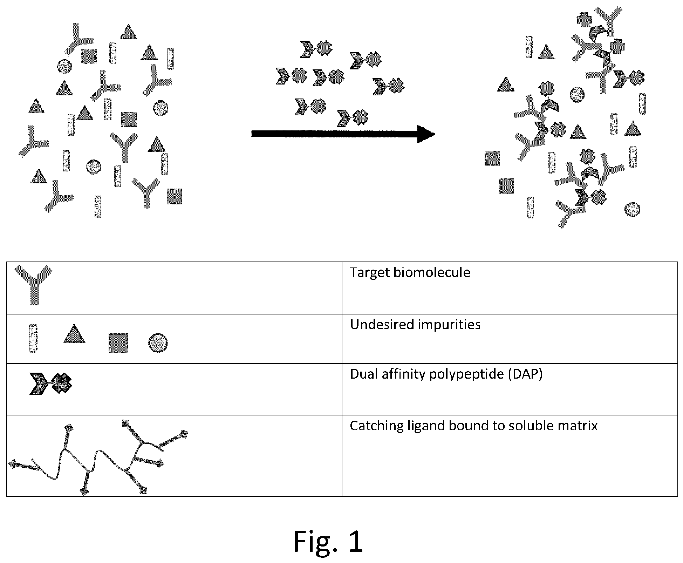

Fig. 1 shows the 3D-complex formation in solution between the target biomolecule and the DAP, i.e., the first step of the claimed method (step a.).

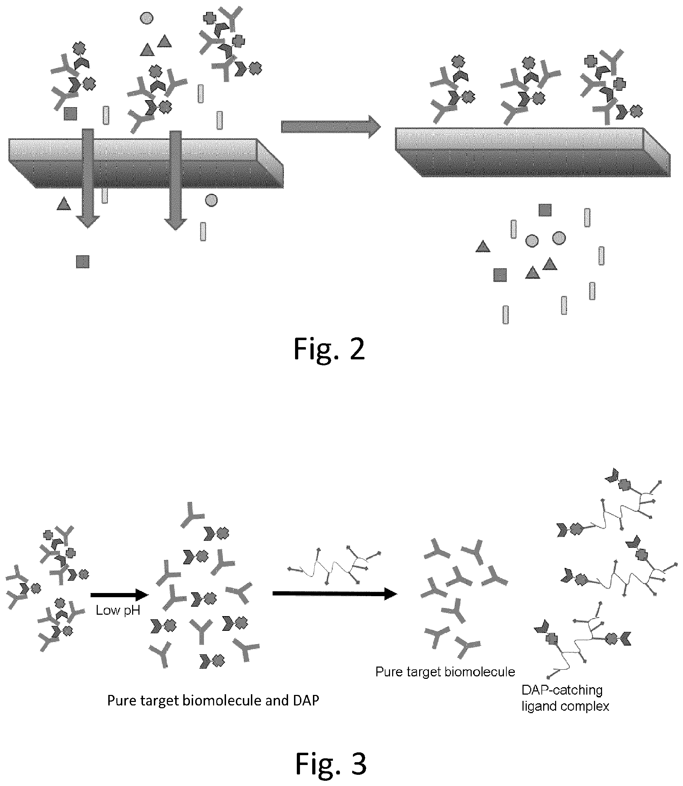

Fig. 2 shows the washing of the formed target-biomolecule-DAP 3D-complexes to remove any impurities and capture of said complexes to obtain a pre-purified target biomolecule-DAP 3D-complex, i.e., the second step of the claimed method (step b.).
Fig. 3 shows the treatment of the pre-purified biomolecule-DAP complex, by dissolution in a buffer composition at a pH below 5.0 to separate the target product biomolecule and the DAP, i.e., the third step of the claimed method (step c.). The resulting mixture is then subjected to a soluble matrix having the catching ligand immobilised to it to remove the DAP from the target biomolecule, i.e., the fourth step of the claimed method (step d.).

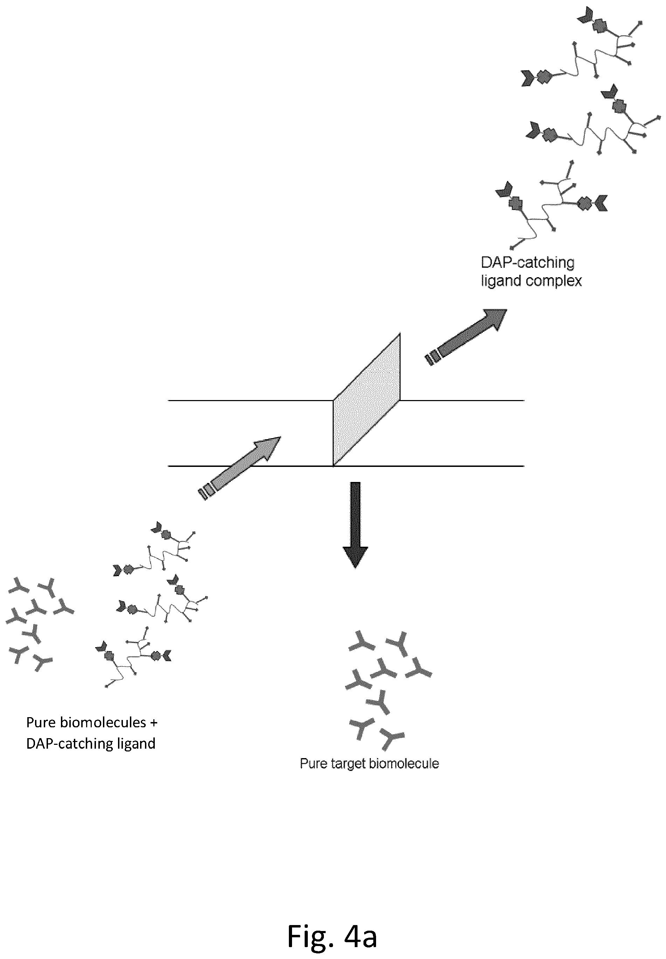

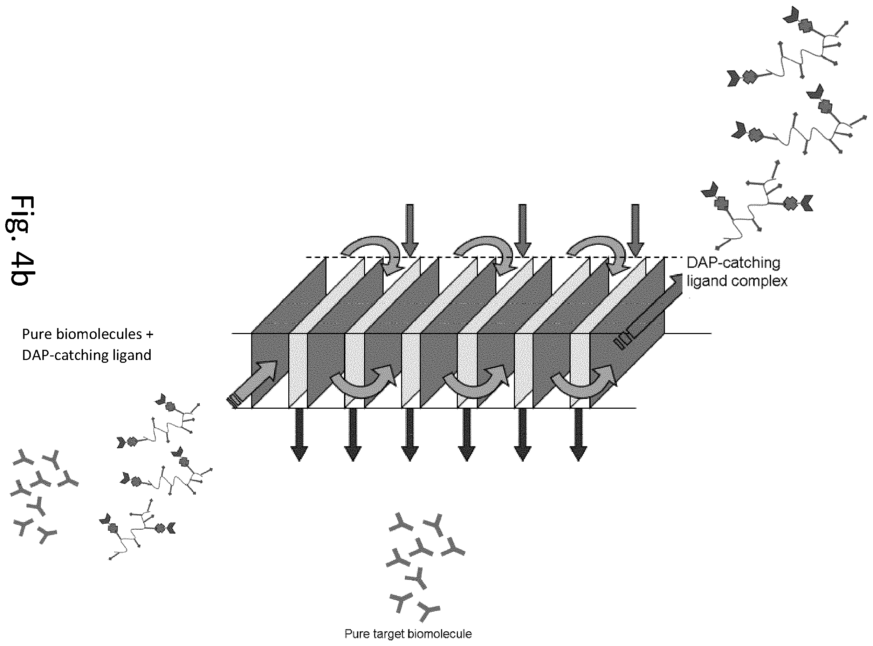

Fig. 4a and 4b shows the recovery of the pure target biomolecule (step e.) using e.g., a single semi-permeable membrane (4a) or e.g., a cross-flow diafiltration system of several semi-permeable membranes in a continuous mode collecting target product biomolecules and removing the DAP-catching ligand-matrix complex (4b).

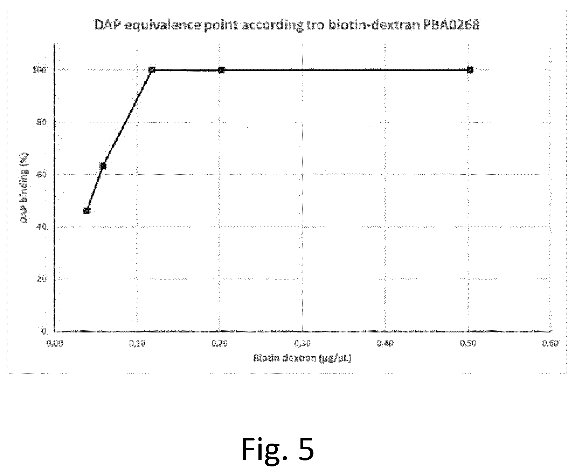

Figure 5: Illustrates the DAP binding (%)/depletion (%) as a function of the biotin-linker dextran concentration. The DAP binding capacity was measured by SEC HPLC by determining the reduction of DAP peak (AUC) as the function of increasing amount of biotin dextran. The curve graph rises to the point of 100% DAP binding (equivalence point) and then stays constant (horizontal part).

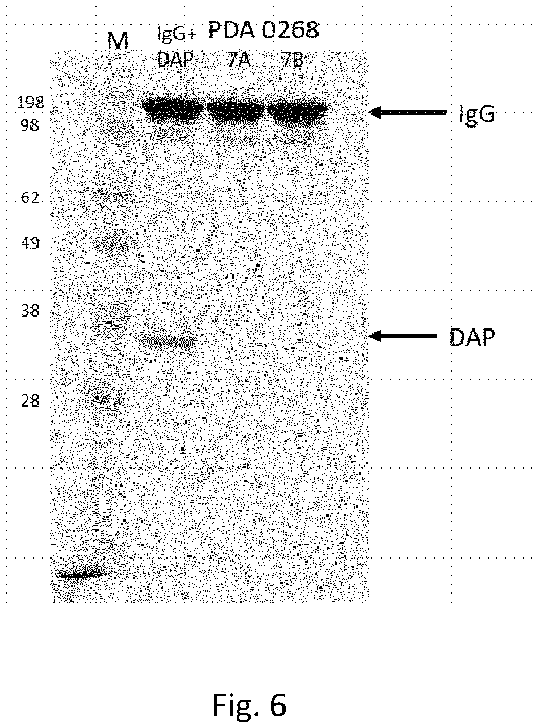

Figure 6: Illustrates the filtrate content in example 11. The SDS-PAGE gel shows the complete DAP removal by biotin-linker soluble matrix PBA 0268 by filtration in C65 depth filter filtration. Lane 1: ("M"): MW marker (10 µl of the Protein Ladder SeeBlueTM Plus2), lane 2 (IgG+DAP): 5 µg Privigen (IgG)+DAP (starting material solution); lane 3 (7a); 5 µg protein loaded from first filtrate fraction and lane 4 (7b): 5 µg protein loaded from filtrate 2. filtrate fraction. No DAP detected in the first to filtrate fractions.

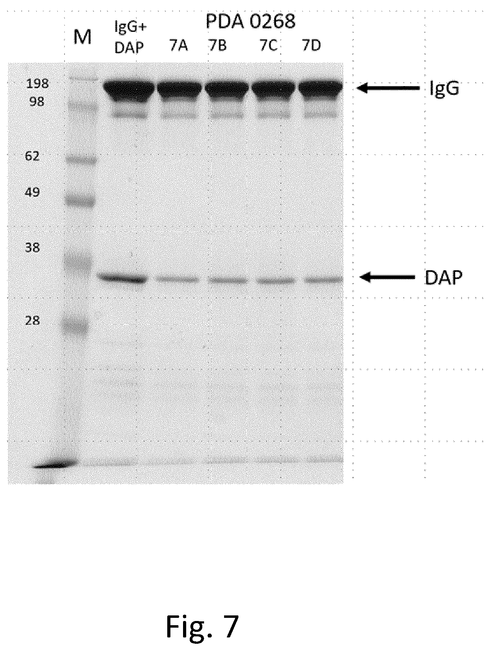

Figure 7: Illustrates the filtrate content in example 13. The SDS-PAGE gel shows the partial DAP removal by biotin-linker soluble matrix PBA 0268. Lane 1: ("M"): MW marker (10 µl of the Protein Ladder SeeBlueTM Plus2, lane 2 (IgG+DAP): 5 µg Privigen (IgG) and DAP (starting material solution); lane 3 (7a); 5 µg protein loaded from first filtrate fraction and lane 4 (7b); 5 µg protein loaded from filtrate 2. filtrate fraction, etc.

### DETAILED DISCLOSURE OF THE INVENTION

The present invention is a radical change from the state-of-the-art affinity purification technology, which utilizes capturing a target on a solid support packed in a column, washing the column and recovering the target by elution.

It should be clear, that the present invention has a different purification workflow: (1) first the target biomolecule in a crude solution is reacted with an added dual affinity polypeptide, the DAP molecule, in solution to form a 3D-complex of target biomolecule and DAP; (2) the target biomolecule-DAP 3D complex is captured by filtration, such as depth filtration, and other impurities are washed away; before (3) the target biomolecule-DAP 3D-complex is dissolved in a buffer solution to separate the target product biomolecule from the DAP; and (4) the DAP is bound to a soluble matrix, removed by filtration and discarded and the purified target biomolecule is collected in solution.

In this process, the added DAP, a purification agent, does not come from the raw or crude material that contains the target biomolecules. The added DAP is an agent for helping the purification process.

After dissolving the DAP-target biomolecule 3D-complex, the mixture only contains the target biomolecule and the DAP. The inventors have realized that it is possible to effectively remove the DAP from the target biomolecule and discard the DAP, by binding the DAP to a soluble matrix, which is easy to remove by e.g., filtering or other unit operation suited for large scale operation.

Binding the DAP molecule to the soluble matrix transforms it into a larger molecule. This transformation increases the dissimilarity in size from the target biomolecule, making it easier to separate the two.

It should be understood that the DAP molecule is not the intended target to be purified in the present affinity purification process. Rather, the intended target is the target biomolecule.

After the target biomolecule-DAP 3D-complex is dissolved (step 3) from e.g., a depth filter, the filter material may be discarded. Also, after the second filtration step, the captured DAP bound to a soluble matrix may be discarded. Consequently, there is no reuse of materials, nor need for cleaning in place procedures at any step in the process.

The purification procedure allows for shorter residence time of the target biomolecule and faster binding reactions in solution. This is of importance for large scale manufacturing, reducing manufacturing cost and optimize effective target product processing.

The core of this invention is the effective, specific and fast removal of the added purification agent, the DAP molecule, by capturing it on a soluble matrix, leaving the pure target biomolecule undisturbed in solution.

In bead-based procedures, cleaning-in-place (CIP) is essential to prevent cross-contamination when repeatedly reusing expensive affinity chromatography beads. Reuse of beads is necessary in order to make the bead-based procedures economical tenable.

The present invention tackles the significant drawbacks of affinity chromatography by introducing a fundamental shift away from bead-based affinity purification. This invention significantly reduces overall processing time, especially under low pH conditions, through rapid elution, efficient DAP removal using a soluble matrix, and effective filtration, followed by pH adjustment. This approach ensures the target biomolecule's quality is maintained at an appropriate pH level.

The speed is achieved through homogeneous binding steps and efficient filtering, and it eliminates the need for costly beads, column packaging procedures to obtain uniform flow and the mandatory CIP procedures and associated quality assurance checks when reused.

Definitions "Purifying", "purification" or other grammatically differing forms of these expressions are intended to mean that the target biomolecule is removed completely or partially from at least one non-target product being present in the starting composition comprising the target biomolecule.

The "target product biomolecule" or "target biomolecule" may in principle be any compound for which a specific binding moiety is known, and which is soluble, preferably in water or aqueous solution. The target biomolecule is preferably a peptide, a polypeptide, an antibody, a virus particle, exosomes (extracellular vesicles), cells or cell components, more preferably an antibody.

The "starting composition comprising the target biomolecule" may in principle be any such composition independently of its origin. As preferred examples of starting compositions comprising the target biomolecule can be mentioned cell-free culture fluids of cell cultures producing the target product biomolecule or any partly purified fraction thereof, or e.g., human plasma.

In case the starting composition comprising the target biomolecule is a culture fluid it is preferred that it is pre-treated before being applied to the method of the invention in order to provide a starting composition comprising the target biomolecule product without any particulate material. Such pre-treatment methods are well-known in the art may be e.g., various conventional filtration techniques or e.g., centrifugation of cell suspensions in case that the target biomolecule is localized extracellularly, or e.g., cell homogenization followed by filtration or centrifugation in case that the target biomolecule is localized intracellularly.

In the case the starting material is human blood, a filtration or centrifugation step may be included prior to carrying out the present invention, to remove the blood cells and isolate the blood plasma component wherein e.g., the target biomolecule is dissolved.

A "dual affinity polypeptide" (DAP) is according to the invention intended to mean a polypeptide having two or more distinct binding sites specific for two or more target molecule binding sites or ligands. A description of DAP and DAP technology can be found in EP 22201017 B1 and EP 2427482 B1. The teaching of these two documents is incorporated in the present application by reference.

The term "first binding functionality" is according to the invention intended to refer to a binding functionality having affinity for the target biomolecule or a group or moiety of the target biomolecule, which is bound by the first binding site of the DAP. The affinity of the first binding functionality of the DAP should be sufficiently high to allow a specific binding of the target biomolecule to the DAP. The equilibrium dissociation constant, K_{D,t} of the DAP to the target biomolecule is preferably in the range of 10⁻⁸ - 10⁻⁴, more preferred 10⁻⁷ - 10⁻⁵, and most preferred around 10⁻⁶.

In the context of the present invention, the equilibrium dissociation constant is measured according to the reaction:

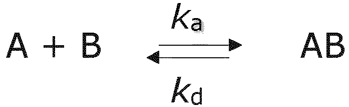

A and B represents the binding partners: the target biomolecule and the dual affinity polypeptide or the dual affinity polypeptide and the catching ligand covalently coupled to a soluble matrix.

The rate constants for the reaction above represent the rate at which the two molecules A and B associates and dissociates: $- \frac{d \left[AB\right]}{d t} = {k}_{d} \left[AB\right]$ $\frac{d \left[AB\right]}{d t} = {k}_{a} \left[A\right] \left[B\right]$

When the rates are equal at equilibrium ka[A][B] = kd[AB], which gives: $\frac{{k}_{d}}{{k}_{a}} = \frac{\left[A\right] \left[B\right]}{\left[AB\right]} = {K}_{D}$ $\frac{{k}_{a}}{{k}_{d}} = \frac{\left[AB\right]}{\left[A\right] \left[B\right]} = {K}_{A}$

The term "second binding functionality" within the context of present invention refers to a binding functionality of the DAP having a high binding affinity for a separate, second ligand, such as for a catching ligand. The DAP according to the present invention has a first binding functionality, which as affinity for the target product biomolecule and another, second binding functionality (i.e., another binding site), which has affinity for the catching ligand covalently coupled to the matrix.

The "catching ligand" is according to the invention intended to mean a ligand different from the target product biomolecule and which is bound by the "second binding functionality" of the DAP. The catching ligand is according to the present invention covalently attached to a soluble matrix. The catching ligand according to the invention may also be covalently attached to a soluble matrix via a linker. The catching ligand may in principle be any moiety or group having the ability to be specifically bound by/to the DAP. The affinity of the catching ligand to the DAP should also be sufficiently high to allow an efficient binding of the DAP to the soluble matrix. The equilibrium dissociation constant, K_{D,s} of the DAP to the catching ligand is preferably in the range of 10⁻¹⁶ - 10⁻¹⁰, more preferred 10⁻¹⁵ - 10⁻¹¹, and most preferred in the range of around 10⁻¹⁴ - 10⁻¹².

The ratio of the binding coefficient of the first ligand to DAP to the binding coefficient of the second ligand to DAP, [K_{D,t}/K_{D,s}], is preferably at least 10, such as more than 10, preferably more than 100, more preferably more than 1000, more preferred more than 5000, even more preferred more than 10000, and most preferred more than 20000.

The term "3D-complex with the target biomolecule" is intended to mean a 3-dimensional complex formed by the binding reaction between the target biomolecule and the DAP.

The term "pre-purified biomolecule" is intended to mean a mixture of target biomolecule-DAP 3D-complexes present in solution without any other impurities being present.

The term "dissolve" or "dissolution" of a target biomolecule-DAP complex refers to the step of treating a target biomolecule-DAP complex in a buffer solution in order to separate the target product biomolecule from the DAP to obtain a mixture of target biomolecules and DAP.

The term "mixture of target biomolecules and DAP" is intended to mean a two-component mixture of target biomolecules in solution, in the presence of DAP. The target biomolecules at this stage are no longer bound or connected to the DAP molecules, therefore DAP may be seen as an impurity in the mixture, which is to be subsequently removed by the subsequent steps of the method of the invention and result in a pure target product biomolecule.

The final filtration step is performed on a solution containing only buffer components, the target biomolecule, and DAP bound to the soluble matrix. The primary objective for the final filtration step is to be rapid, gentle and not interacting with the target biomolecule. Ideally, the target biomolecule should remain largely unaffected during the removal of the DAP-soluble matrix complex.

The critical characteristics of the soluble matrix include water solubility or solubility in solutions with over 50% water content, availability in industrial-scale quantities, ease of modification with binding ligands, and being non-toxic. To enhance the reaction rate of binding, the soluble matrix should be in a non-solid state, not in the form of amorphous particles, and should exist as molecules in a nearly perfect solution. This characteristic allows for effective mixing, convection, and consequently fast binding of DAP to the matrix in the solution.

In an embodiment of the invention the soluble matrix is a polymer.

Non-limiting examples of suitable polymers include dextran, xanthan gum, pectin, chitin and chitosan, carrageenan, guar gum, cellulose ethers, hyaluronic acid, albumin, hydroxy ethyl starch and other starch derivatives, polyacrylic acid, polyacrylamides, polyvinyl alcohols, polyethylene glycols, polyvinyl pyrrolidone, divinyl ether-maleic anhydride, polyoxazoline, polyphosphates, polyphosphazenes, copolymers thereof, and mixtures thereof. Preferably, the matrix is a polymer selected from the group consisting of dextrans, carrageenan, pectins, cellulose ethers, polyacrylic acid, polyacrylamides, polyvinyl alcohols, copolymers thereof, and mixtures thereof and even more preferably, the matrix is a dextran polymer.

The soluble matrix in the form of a polymer may be partly crosslinked to increase the overall molecular weight and change the molecular size, flexibility and density, to an extent that it is still soluble.

Filtering in this context is the separation process, that separates and removes the matrix with the bound DAP from the target biomolecules.

Filters have extensive applications in water treatment, food processing (including dairy, brewing, and juice production), chemical processes, and the life science sector, particularly in downstream processing of recombinant proteins, polishing steps, and viral removal.

Various filtering processes are commonly understood within the domain of chemical and bioprocessing unit operations. Filtering is extensively employed across industrial applications and encompasses diverse methods, such as crude particle filters, asymmetrical depth filters, depth filters or dead-end filters with filter aids, and nano, ultra and microfiltration using specialized membranes. It's important to note that the terms "filter" and "membrane," as well as "filtering" and "membrane filtering," are used interchangeably in this context.

The membranes can be in the form of e.g., flat sheets, hollow fibres, and arranged in e.g., plate & frame modules or spirals in housing with single or multiple chambers.

Filtering systems, whether operated in batches or continuous, offer versatile options. They can incorporate controlled flow and pressure, use cross or counter flow arrangements, traditional or single-pass tangential flow filtration, with or without recycling and optimize factors like target biomolecule collection speed, residence time, fouling, energy consumption, and operational costs.

Other advanced filter systems are well-known to industry experts, such as large-scale setups with automatic scraper strainers, overflow and backflow configurations, stacked filters, variable filter sizes, and automated monitoring and control of flow speeds and pressures. These combinations are readily available in industrial applications.

Considering that the target biomolecule is in the eluate while the DAP-Matrix is retained, continuous filtering operations are preferred. This choice accommodates reducing downstream processing volumes, reduces residence time, and minimizes the environmental and physical footprint of the operation.

Industrial filtering employs a wide array of materials in varying shapes and properties, from basic options like raw paper, cloth, polymers, and glass-fiber matrices to advanced polymeric membranes with precise pore structures and permeability properties, such as ceramics, polysulfone, regenerated cellulose, cellulose acetate, nitrocellulose, cellulose esters, polysulfone, polyether sulfone, polyacrylonitrile, polyamide, polyimide, polyethylene, polypropylene, polytetrafluoroethylene, polyvinylidene fluoride, polyvinylchloride, and their blends.

Filter aid is used in e.g., depth filter operations. Filter aid is typically a low-density, fibrous, or fine granular material used to prevent fouling, clogging of the filter, increase the flow rate and improve the operational performance and quality of filtration.

Filter aids are used to pack the depth filter that improve the permeability and sometimes porosity of a filter cake, improve filtrate clarity and help to prevent filter medium blinding or filter clogging. They comprise relatively porous particles such as diatomaceous earth, perlite, celite, kieselguhr or activated carbon and are filtered as a precoat onto the medium or alternatively, mixed as body feed with the solution or suspension during a pretreatment stage.

In most filtering processes, the mechanism for separation relies on a combination of size and interactions with the filter membrane, filter aid or system. This means it is often challenging to clearly differentiate between size-based separation and other factors like ionic or hydrophobic interactions, when molecules pass through a filter or membrane.

The inventors have recognized the significance of both the size and size distribution of the soluble matrix. A more substantial disparity in size between the target biomolecule and the DAP-matrix simplifies the filtration process.

Polymer or polymer populations typically exhibit a size distribution and an average molecular weight. In this context, the average molecular weight is less critical compared to, for example, the average size of the smallest 10% of the distribution. A broader distribution implies a higher proportion of smaller molecules in the population.

Therefore, it is preferable to apply a matrix, including the DAP-polymer, with a narrow molecular weight distribution and a sufficiently large average size to optimize the filtering separation's effectiveness. Even more preferred use is made of a polymer population from which the smallest molecules have been removed. Such a polymer can be characterized by both the average molecular weight and the weight interval for e.g., the lowest 10% of the population.

To eliminate the smallest molecules, techniques like crossflow filtration with an appropriate membrane cut-off can be employed. This results in a polymer matrix population with no sizes falling below a molecular cut-off value. It is important to note that the amount of DAP binding to the matrix increases the average molecular size and broadens the size distribution of the DAP-matrix population.

The ultimate goal is to establish a substantial size difference between the DAP-matrix and the target biomolecule, ensuring that all DAP-matrix molecules within the population are distinctly larger than the target biomolecule. This facilitates a swift and efficient separation by filtration.

The core of the invention is that by adding the soluble matrix to the solution containing target biomolecule and DAP purification agent, the DAP is bound to the matrix, which is transformed into a much larger molecule with distinctly different properties compared to the target biomolecule. The matrix-bound DAP can be regarded as a very large polymeric DAP molecule, including the specific properties of the DAP, including hydrophobic, ionic or other physical or chemical properties.

The selection of effective filtration methods is well-known for the skilled person in the art. A solution of target biomolecule and DAP bound to the soluble matrix is pumped into a filter system and the target biomolecule and the DAP-bound matrix is separated and measured in the retentate, filtrate, respectively. By changing e.g., filter type and arrangement, filter aid type and volume or membrane type or area, pressure, recycling and flowrates the performance can be fine-tuned. The objective is to find the conditions, where the target biomolecule is recovered and the DAP-matrix is removed as much as possible. Preferably, the DAP content in the filtrate is less than 1%, more preferably less than 100 ppm, even more preferably less than 5 ppm.

Surprisingly, it was discovered that both a membrane system and a traditional depth filter with filter aid proved highly effective in separating the DAP bound to the matrix from the target biomolecule. The target biomolecule was successfully recovered in the filtrate, while the DAP bound to the matrix was retained in the retentate or within the depth filter matrix and could be subsequently discarded.

### Specific embodiments of the invention

The method according to the invention provides reduced reaction and incubation time, which results in an increased productivity. The process is straightforward to scale up. Further, the method described reduces the production cost and allows for implementation in industrial scale

### Step a. of the method - Fig. 1

In the first step of the method of the invention the starting composition comprising the target biomolecule is contacted with a DAP having affinity for the target biomolecule in solution, and the mixture of the starting composition comprising the target biomolecule and the DAP is maintained in solution for a time suitable for formation of 3D-complexes of DAP and the target biomolecule. The target biomolecule may be e.g., a protein, or preferably an antibody, a virus particle, an exosome or cells or cell components. As the binding reaction is rapid in solution the contacting may in principle be done using any procedure and equipment capable of efficient mixing two liquids, including e.g. both traditional mixers and static mixers. Such an operation is well-known, and the skilled person will appreciate to select suitable equipment and conditions for this step.

The 3D-complexes are formed immediately after mixing the components and therefore no need to include a long incubation time for 3D-complex formation.

### Step b. of the method - Fig. 2

The 3D-complexes formed in step a. of the process are washed to remove any impurities and to form a pre-purified target biomolecule-DAP 3D-complex. The washing is preferably performed by filtration, such as via depth filtration. A filter aid may be used establish a depth filter to improve filtering capacity and efficiency.

Filtering is possible as the 3D-complexes formed in step a. are partially insoluble, forming a turbid suspension.

A buffer may be used to wash the captured biomolecule-DAP 3D-complex and to wash off the impurities.

### Step c. of the method - Fig. 3

In a third step, the target biomolecule is dissolved from the DAP, i.e., the target biomolecule-DAP bond is broken using a method depending on the particular properties of the binding sites of the target biomolecule and DAP. For example, a buffer may be used to treat the pre-purified target biomolecule-DAP 3D-complex. The 3D-complex is therefore dissolved in a buffer, which detaches the target product from the DAP. The buffer may be the same buffer used in the second step, or the buffer may be a different buffer than the buffer used in the second step. The solution now only contains the target biomolecules and the DAP.

Preferably, the target biomolecule-DAP-3D-complex may be dissolved into target biomolecule and DAP by changing the pH in the elution buffer and more preferably, by lowering the pH below 5.0. Preferably, the pH in step c. is in the range 2.8-4.7, preferably in the pH range 3.1-4.5, more preferably in the pH range of about 3.4-4.3.

### Step d. of the method -Fig. 3

In the next step the mixture comprising the target biomolecules and the DAP is contacted with a soluble matrix having the catching ligand covalently bound to it to form target biomolecules and DAP-catching ligand-matrix products in solution. In this step only the DAP will bind to the soluble matrix by the second binding functionality and the target biomolecules will stay unaffected in the solution.

The soluble matrix may be a water-soluble matrix or is soluble in an aqueous solution. However, other solvents may also be used e.g., organic solvents, as long as the solvent is compatible with the target biomolecule and the matrix. The matrix may be a linear, nonlinear, branched or crosslinked matrix.

The soluble matrix may be a polymer selected from the group consisting of dextrans, polyacrylic acid, polyacrylamides, polyvinyl alcohols, polyethylene glycols, copolymers thereof, and mixtures thereof. Preferably, the matrix is a dextran polymer.

The polymer may have an average molecular weight (Mw) of 200-5.000 kDa. Preferably the polymer has an average molecular weight (Mw) of 200-5.000 kDa, where the smallest 10% fraction in the distribution is more than 200 kDa. Even more preferably, the polymer has an average molecular weight (Mw) of 500-4.000 kDa, where the smallest 10% fraction in the distribution is more than 500 kDa.

The dextran polymer may have an average molecular weight (Mw) of 200-5.000 kDa. Preferably the dextran polymer has an average molecular weight (Mw) of 200-5.000 kDa, where the smallest 10% fraction in the distribution is more than 200 kDa. Even more preferably, the dextran polymer has an average molecular weight (Mw) of 500-4.000 kDa, where the smallest 10% fraction in the distribution is more than 500 kDa.

Optionally, the difference in size, such as the difference in average molecular weight (Mw) between the matrix and that of the target biomolecule, is at least 3 times, such as a difference of at least 5 times, such as at least 7 times, such as at least 10 times. The size and size distribution can be estimated by numerous methods, including e.g., size exclusion chromatography, dynamic light scattering, hydrodynamic chromatography and field-flow fractionation.

In order to provide an efficient recovery of the pure target biomolecules, the difference in size, such as the difference in average molecular weight (Mw) between the DAP-soluble matrix and that of the target biomolecule is at least 3 times, such as a difference of at least 5 times, such as at least 7 times, such as at least 10 times. The molecular size may be described in terms of Stokes radii.

The matrix may be connected to the catching ligand via a linker. However, the catching ligand may be directly connected to the matrix without a linker, using conjugation chemistry.

In case a linker molecule is used to create space between the soluble matrix and the catching ligand, the linker is covalently attached to the soluble matrix using conjugation chemistry. Preferred methods for covalently attaching the linker to the soluble matrix may be e.g., coupling amino functionalized capturing linker with carboxyl modified dextran using carbodiimide or an active ester.

In case the matrix is connected to the catching ligand via a linker molecule, the linker preferably has a length of 10-25 bonds, such as a length of 10-24 bonds, such as a length of 10-23 bonds, such as a length of 10-22 bonds, such as a length of 10-21 bonds, such as a length of 10-20 bonds, such as a length of 10-19 bonds, such as a length of 10-18 bonds, such as a length of 10-17 bonds, such as a length of 10-16 bonds, such as a length of 10-15 bonds, such as a length of 10-14 bonds, such as a length of 10-13 bonds, such as a length of 10-12 bonds, such as a length of 10-11 bonds, such as a length of 11-25 bonds, such as a length of 12-25 bonds, such as a length of 13-25 bonds, such as a length of 14-25 bonds, such as a length of 15-25 bonds, such as a length of 16-25 bonds, such as a length of 17-25 bonds, such as a length of 18-25 bonds, such as a length of 19-25 bonds, such as a length of 20-25 bonds, such as a length of 21-25 bonds, such as a length of 22-25 bonds, such as a length of 23-25 bonds, such as a length of 24-25 bonds.

The linker may also be a peptide.

The linker may be selected from the group consisting of 2-Vinyl-4,4-dimethyl-5-oxazolone (VDMA), vinyl azlactone derivatives, acrylic derivatives, hexandiisocyanate (HDI) derivatives, diisocyanate derivatives, or mixtures hereof.

The catching ligand may be selected from the group consisting of biotin, and biotin analogues. Preferably, the linker is Biotin-C17-NH₂. 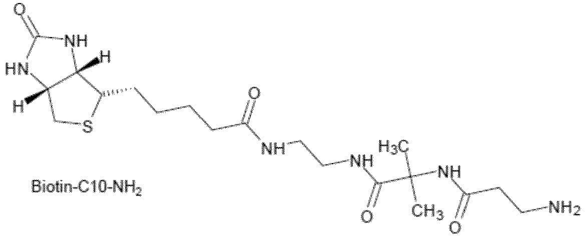
Structure of Biotin-C10-NH₂. 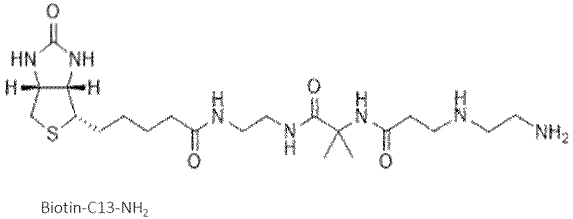
Structure of Biotin-C13-NH₂. 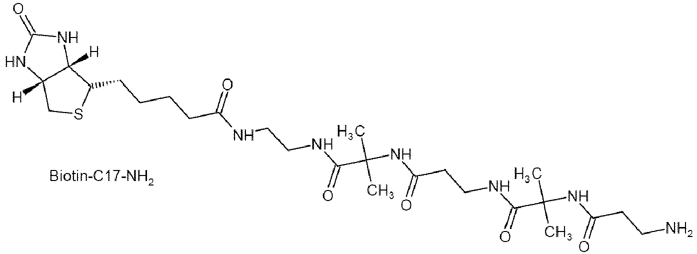
Structure of Biotin-C17-NH₂. 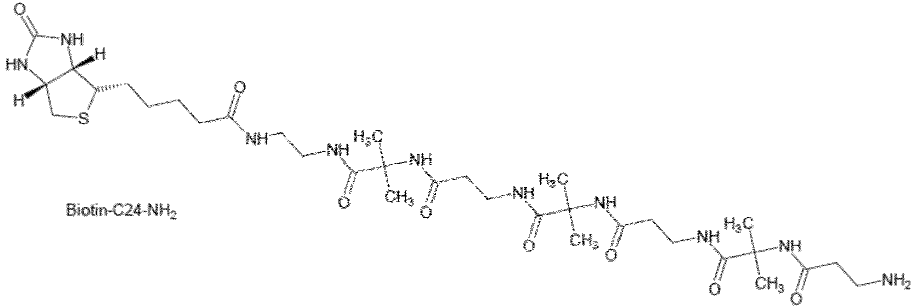
Structure of Biotin-C24-NH₂.

### Step e. of the method - Figs. 4a & 4b

In a last step, the DAP-ligand matrix is removed from target biomolecule in solution.

Preferably, the separation of the target biomolecule and the DAP-catching ligand-matrix products are by filtration, such as by membrane filtration or depth filtration. Preferably, the filtration allows the passage of the pure target biomolecule and retains the DAP-catching ligand-matrix products. This type of filtration is a well know procedure for individuals skilled in the art and examples are e.g., filtration using semi-permeable membranes or depth filtration established using filter aids.

Membrane filtration techniques are particularly useful in the method of the invention using membranes permeable to the pure target biomolecule but impermeable to the complex consisting of separate DAP-catching ligand-matrix. Preferably, permeable filters are semipermeable membranes. Preferably, the recovery is performed using filtration, such as diafiltration, cross-flow diafiltration, hollow fibre filtration, microfiltration or ultrafiltration.

A particularly preferred separation process is continuous cross-flow filtration, in particular cross-flow diafiltration, wherein the product stream comprising the complex of separate DAP-catching ligand-matrix and the target biomolecule is flowing through membranes impermeable for this complex but permeable to the target biomolecule. The washing filtration can be carried on until the target biomolecule is collected in the filtrate and the retentate practically comprises only compounds that are not able to pass the membrane, i.e., separate DAP-catching ligand-matrix.

Various membranes can be selected depending on the size of the target biomolecule. Preferably, the membrane is selected to allow the target molecule to pass through the membrane while retaining the DAP-catching ligand-matrix products.

Suitable membranes typically have a cut-off value of >50kDa, preferably >100kDa, more preferably >150kDa, even more preferably >300kDa or >500kDa depending on the DAP-catching ligand-matrix and target biomolecule in question.

When the target biomolecule is e.g. IgG, suitable membranes have a typical cut-off value of >150kDa, suitably >300kDa, more suitably >500kDa. When the target biomolecule is e.g. albumin, suitable membranes have a typical cut-off value of >50kDa, suitably >100kDa.

Another preferred method for carrying out the separation and/or recovery step is by using hollow fibre filtration, wherein the product stream is led inside hollow fibres made of a semi-permeable material that is impermeable to DAP-catching ligand-matrix but permeable for the target biomolecule. An exemplary recovery process is shown on Fig. 4a and 4b.

The membranes can be arranged as dead-end or depth filters with filter aid as previously described or as hybrid systems which combines the filtration types, like 3M Emphaze hybrid purifier filter or with a continuous flow across the membrane to avoid fouling and to control the pressure more easily. Examples include diafiltration, crossflow filtration, hollow fibre systems etc. The membranes can be stacked together, arranged in spirals, plate and frame or hollow fibres or in cascade arrangements (see continuous arrangement, figure 4B). Pressure may or may not be applied during the filtration process. Typically, the filtrate passing through the membrane comprises the target biomolecule and the DAP-catching ligand-matrix is retained in the retentate. The retentate may be recycled into the feed stream to increase separation yields. The retentate may also be discarded without recycling.

The flux through the membrane, and consequently the speed of membrane filtration, depends largely on factors such as the volume processed, membrane area, pore sizes and distribution, pressure, and the potential for fouling. Utilizing a larger filter area, for instance, can reduce the filtration time significantly.

Typically, the residence time in the membrane system is short e.g., at most 60 minutes, at most 50 minutes, at most 40 minutes, at most 30 minutes, at most 20 minutes, at most 15 minutes, at most 10 minutes, at most 5 minutes, however the membrane system can be further optimised to fit to the specific procedure.

It is appreciated that other suitable membrane filtration systems may be used without departing from the scope of the invention, i.e. different types of membranes with suitable size cut-offs may be used, arranged in series and with recycled streams to optimise the flow rate, residence time and separation efficiency.

After the recovery of the target biomolecule, a suitable buffer may be added to the target biomolecule solution to adjust the pH and salts conditions to stabilise the pure target biomolecule. Preferably, the pH is raised to near neutral pH. Even more preferably, for IgG purified from plasma, the pH is adjusted to 4-5. However, the pH may be raised to other suitable pH most suited for preserving the stability and quality of the particular target molecule.

After this step, the pure target product may optionally be further polished and filtered to remove any leftover impurities, e.g., viral particles etc.

After the purification method of the invention the target biomolecule may be formulated as desired using techniques well known in the art. The DAP-catching ligand-matrix may be discarded.

The purification method according to the invention is fast due to the optimized mass transfer processes in solution (mass transport by convection not by diffusion as in conventional affinity purification with solid resin-based systems). The DAP may be fermented in a large-scale production facility to become a low-cost item. Exemplary DAP production methods are disclosed in EP 2220107 B1. The process of the invention improves the overall production efficiency both economically, environmentally and timewise.

### EXAMPLE 1

*General procedure for synthesis of carboxy methyl dextran 500 (CM-Dextran 500). Example is for substitution of every 15-30 Glucose unit.*

Dextran (T500, Pharmacosmos A/S), KOH (Fluka) and chloroacetic acid solutions was prepared. Chloroacetic acid solution was also freshly prepared: chloroacetic acid (Aldrich, 9.45 g; MW 94.49 g/mol) added to Type 1 water (75 ml) on ice bath, Na2CO3 (4.77 g; 0.9 mol equiv.) was added.

Slowly 25 ml of the 4 N KOH solution was added to 50 ml of 10% Dextran 500. Then 25 ml of 1 M Chloroacetic acid solution was added under gentle stirring. The reaction mixture (in total, 100 ml, 5 % Dextran, 0.25 mol chloroacetic acid and 1 mol KOH) was kept for 1 h at 65 °C in water bath. The solution was stirred gently from time to time.

The reaction was stopped by adjusting the solution to pH 3.0, by gently adding 1 M HCl. The solution was kept cold (below room temperature) and the CM Dextran was precipitated by adding the cold solution very slowly to 500 ml methanol. The CM Dextran 500 was collected by a suction Buchner funnel and washed with cold methanol (20 ml) twice. The collected CM-Dextran was. vacuum dried overnight and the collected CM-Dextran was weighted. The precipitation procedure was repeated by re-precipitation of CM Dextran 500, by re-dissolving CM-Dextran in a few ml of 5 mM KOH and re-precipitating by adding the KOH solution of Dextran 500 slowly into cooled methanol (5 x the CM-Dextran 500 in 5 mM KOH). Characterization was carried out by simple pH titration with pH meter (Metler). Approximately every 15-30 Glucose unit is of Dextran modified by carboxymethyl groups.

### EXAMPLE 2

Coupling of Biotin-C13-linker-NH2 to CM-Dextran 500 via N-Hydroxy succinimide ester (NHS) pre-activation. The following example given for 1/25 equivalent linker modification. The addition of Biotin-C13-linker-NH2 (1/25mol equiv. per Glc unit) to NHS preactivated CM-Dextran 500 in 0.5 M MES pH 6.0, resulting in a biotin linked soluble dextran matrix.

First, EDAC (Aldrich, 59.4 mg, 1/10 mol equiv. per Glc unit) and N-Hydroxy succinimide (NHS) (14,27 mg, 1/25 mol equiv.) was added to CM-Dextran 500 (500 mg, 3.1 mmol Glc units) in order to synthesize "in-situ" the NHS-CM-Dextran. The reaction mixture was kept at room temperature for 2 hours under mixing.

Second, after 2 hours the Biotin-C13-NH2 linker (1-25 mol equiv., 485 g/mol, 60,26 mg) in 0.5 ml 0.5 M MES buffer pH 6.0 was added and the mixture was kept overnight at room temperature under mixing in a "blood sample mixer". The Biotin-C13-NHCO-CH2-Dextran 500 (1-25 mol equiv.) was purified by precipitation in methanol (ratio 5:1), filtration and washing with cold MeOH (5x 5 ml) and cold acetone (5x5ml). The precipitated Biotin-C13-linkerNHCO-Dextran 500 was then collected of by vacuum filtration on a Buchner funnel, washed with 5x 5 ml cooled methanol and 5x cold acetone and was then vacuum dried in a desiccator. The powder was then dried "in vacuum" in a desiccator overnight.

Dextrans with 1/25, 1/50 and 1/100 equivalent biotin linker per CM dextran 500 were produced.

### EXAMPLE 3

### The equivalence point determination for DAP molecules in solution against biotin linked soluble dextran matrix, as determined by SEC-HPLC

A fixed amount of DAP molecule (2.13 mg/mL stock solution of the DAP) was diluted to 0.80 mg/ml and was titrated against known dilution series of the soluble capture matrix in microtiter plate wells (Dex 1-13). The recombinant a dual affinity polypeptide (DAP) molecule consists of multiple binding domains from Protein A fused to Streptavidin. The DAP is expressed by an E. coli cell line and the purified protein was obtained from CHRETO Aps, Denmark.

Samples was analyzed by SEC-HPLC (TSKgel 300 column, Tosoh Bioscience) and the area under the curves (AUC) from the free DAP, the matrix and the DAP Matrix recorded.

The various retention time and AUC is summarized in Table 1 below.

**Table 1. Absorption measurement data: soluble biotin linker dextran matrix titrated against DAP. AUC is the area under the curve.**

| Vial # | Sample Name | Biotin-linker Conc | DAP conc. | Retention time (Dex 500-DAP) | AUC (Dex50 0-DAP) | AUC (DAP) | AUC total | DAP depletion (%) |
|---|---|---|---|---|---|---|---|---|
| 1 | Buffer Blank | 0.0 | 0.00 | | | | | |
| 2 | DAP blank | 0.0 | 0.80 | | | 362.469 | | |
| 2 | DAP blank | 0.0 | 0.80 | | | 405.502 | | PBA02 35 |
| 3 | Dx-1 | 0.0469 | 0.80 | 6.097 | 451.715 | 0 | 451.7 15 | 100.0 |
| 4 | Dx-2 | 0.0391 | 0.80 | 6.096 | 464.364 | 0 | 464.3 64 | 100.0 |
| 5 | Dx-3 | 0.0313 | 0.80 | 6.086 | 456.953 | 0 | 456.9 53 | 100.0 |
| 6 | Dx-4 | 0.0247 | 0.80 | 6.065 | 450.152 | 0 | 450.1 52 | 100.0 |
| 7 | Dx-5 | 0.0201 | 0.80 | 6.086 | 431.183 | 0 | 431.1 83 | 100.0 |
| 8 | Dx-6 | 0.0156 | 0.80 | 6.107 | 351.275 | 55.445 | 406.7 20 | 86.4 |
| 9 | Dx-7 | 0.0117 | 0.80 | 6.114 | 275.634 | 135.182 | 410.8 16 | 67.1 |
| 10 | Dx-8 | 0.0078 | 0.80 | 6.118 | 187.791 | 223.800 | 411.5 91 | 45.6 |
| 11 | Dx-9 | 0.0059 | 0.80 | 6.123 | 145.767 | 265.657 | 411.4 24 | 35.4 |
| 12 | Dx-10 | 0.0047 | 0.80 | 6.122 | 112.677 | 305.617 | 418.2 94 | 26.9 |
| 13 | Dx-11 | 0.0039 | 0.80 | 6.123 | 98.423 | 323.332 | 421.7 55 | 23.3 |
| 14 | Dx-12 | 0.0029 | 0.80 | 6.118 | 76.092 | 345.093 | 421.1 85 | 18.1 |
| 15 | Dx-13 | 0.0014 | 0.80 | 6.121 | 38.653 | 377.999 | 416.6 52 | 9.3 |
| 16 | C13-dx-blank | | 0.00 | 6.109 | 297.985 | Mean | | |
| 17 | PBA0235 (2x dil.) | | 0.000 | 6.357 | 10.787. 316 | | | |
| 18 | PBA0235 (4x dil.) | | 0.000 | 6.224 | 5.285.8 46 | 21.359. 008 | | |

### EXAMPLE 4

The basic approach for purifying the target molecule is illustrated below for the purification of IgG from plasma, using DAP and the soluble capture matrix, the biotin linker dextran matrix.

The recombinant DAP was prepared as explained in e.g. EP2427482. The recombinant DAP molecule consists of binding domains from Protein A fused to Streptavidin. Plasma sample was a gift from the local municipal blood bank.

The plasma solution (46 mL, 6.5 g IgG/L) was mixed on a static mixer with a mixture of DAP solution (42.7 mg DAP, 6.1 ml) and 40 ml reaction buffer (0.1 M Na-phosphate buffer, 0.15 M NaCl, 0.1% Tween-20, pH 7.2) and was incubated for 5 minutes with a static mixer.

In a first alternative, the suspension of formed DAP-IgG complexes was added to 6g filter aid (Celpure C65 filter aid from Advanced Minerals, bought through Filtrox AG, Sct.Gallen, Switzerland) and collected on depth filter and was washed three times (50 ml) with different wash buffers: Wash buffer 1: 0.1 M Na-phosphate buffer, 0.15 M NaCl, 0.1% Tween-20, pH 7.2.
Wash buffer 2: 0.1 M Tris-HCl + 2 M NaCl, pH 7.4,
Wash buffer 3: 50 mM Tris-HCl, pH 7.4. Followed by 50 mL type 1 water.

In a second alternative, the suspension of formed DAP-IgG 3D complexes was mixed with diatomite high-purity filter aid (HPFA) (6g, Celpure C65, Advanced Minerals/Imerys) and established as a depth filter and repeatedly washed with different wash buffers (50 mL), until other plasma proteins were completely washed away from the uniform filter cake, according to SDS-PAGE analysis: Standard Tris buffer, pH 7.4., Phosphate/NaCl with or without 0.1% Tween20, pH 7.2, and finally with type 1 MilliQ water.

The IgG-DAP 3D complex was dissolved in an elution buffer (0.1 M Citrate, 0.03 % Tween-20, pH 3.4).

The mixture of IgG and DAP (0.80 mg/ml) was then contacted with a soluble capture matrix having a catching ligand bound to it, Biotin-C13(linker)-Dextran 500 matrix (Equivalent to approx. 0.0201 mg biotin/ml). After 2 minutes incubation, the free target IgG was separated from DAP-catching ligand-matrix and collected as the permeate from a Merck Pelicon cassette XL50 with 1000 kDa Millipore membrane.

In the following, soluble matrix materials made of larger dextrans with controlled molecular weight and molecular weight distribution were prepared, functionalized with chloroacetic acid, coupled with a biotin linker and tested for capturing a dual affinity polypeptide (DAP) and remove the same by filtering, using depth- or membrane filters.

Numerous soluble matrixes and combinations were prepared to illustrate the utility and possible variations of the method according to the invention. Also, the following examples describe the systematic optimization of the individual unit operations, by only changing few parameters for each step. It should be clear, that a person skilled in the art of conjugation and filtration unit operations can further change the various parameters.

### Example 5

### High molecular weight Dextran purification:

Various large dextrans with reduced amount of low molecular weight chains or narrow molecular weight distribution was received from PK Chemicals A/S, Køge, Denmark or Pharmacosmos A/S, Holbaek. The average size and distribution of the dextrans were measured by size exclusion chromatography (phosphate buffer pH 7, flow 0,6 ml/min. 2,50 mg/ml, Waters Ultra hydrogel Linear 7,8x300 mm), and using a refractive index (Waters) detector for concentration and online multi-angle light scattering (MALS) size detectors (three laser MiniDawn Treos, Wyatt Technology Corp).

Examples of both technical quality and high molecular weight dextrans washed 4 times in 100 kDa cut-off membrane systems are listed in the table below, with the average molecular weight size and the average molecular weight for the lowest and highest 10% fractions.

**Table 2: Technical quality and filtered high molecular weight dextrans.**

| No | ID | Source | Filtered | Average Mw, kDa | Low Mw, kDa, 10% fraction | High Mw, kDa, 10% fraction |
|---|---|---|---|---|---|---|
| 1 | U222001 | PKC | Technical | 63 | 38 | 123 |
| 2 | Dex500 | Pharmacosmos | Technical | 494 | 137 | 1952 |
| 3 | U323007 | PKC | Technical | 1169 | 82 | 5622 |
| 4 | U40210 | PKC | 100kDa membrane | 2045 | 332 | 6329 |
| 5 | U223008 | PKC | 100kDa membrane | 1903 | 626 | 5388 |
| 6 | U40306 | PKC | 100kDa membrane | 5072 | 4916 | 8198 |

The example illustrates the possibility to source and prepare very differently sized soluble polymers, in this case dextrans, as basis material for a soluble matrix, and with different molecular weight distributions and cut-offs. The exact selection of the specific sized soluble matrix depends on the subsequent DAP loading per soluble matrix, the specific target biomolecule size, filtration unit operation and required DAP removal criteria.

In the following examples, one of several methods of activation, refining and coupling a catching ligand to a soluble dextran matrix is described.

### Example 6

### CM Dextran preparation and analysis

Various carboxymethyl dextrans (CM-Dextran) were prepared as in example 1, using chloroacetic acid and KOH. After recovery by precipitation in methanol and drying, the acid content was measured by pH titration.

In short, CM-Dextran was dissolved in Type 1 water (10 % (w/w), 10 mL) and a few drops of HCl (1.0 M) were added to make sure the carboxyl acids were all protonated. The CM-Dextran was precipitated by dropwise addition to a magnetically stirred cold methanol solution (5 x volume, 50 ml), collected by filtering in a Buchner funnel and washed with cold methanol, until the methanol run-through was neutral (1:1 dilution in Type 1 water, Mettler-Toledo pH meter). The CM-Dextran was dried in a vacuum desiccator until constant weight. The CM-Dextran was titrated with HCl. The CM-Dextran solution (1.00 % (w/w), 10 ml) was added NaOH (0.010 N, 10 ml) and titrated with a HCl solution (0.010 N) using a burette while recording the pH as a function of added HCl volume to determine the equivalence point. Unmodified dextran was titrated for reference. The equivalence point was calculated from the CM-dextran pH curve in comparison to a dextran pH curve.

Using the exact stoichiometry and conditions as in example 1, the CM loading per gram CM-Dextran was measured to 310-320 µmol carboxyl acids/gram for all sizes and distributions of dextran. This corresponds to a modification of about 6% of the glucose subunits.

By changing the reaction time or temperature, the degree of substitution could be changed.

### Example 7

### Molecular weight refined CM-Dextran

In the following example, a large molecular weight carboxymethyl dextran (CM-Dextran) was further refined to remove chemical reactants and lower molecular weight fractions. In this example, the CM-dextran refined was made from the dextran with average Mw of 1.903.410 KDa (#5, in example 5).

In short, a tangential flow filtration system (Minimate EVO TFF, Pall, mounted with three different MW cut-offs membrane capsules of 50 cm² effective filtration area: Minimate^{™} Tangential Flow Filtration Capsules, Pall, 100K (OA100C12), 300K (OA300C12) and 500K (OA500C12).

In summary, for each of the three membranes, a 2% solution of CM Dextran (in Type 1 water, 50 ml) was loaded to the reservoir of the system. The system was operated at 1 bar pressure to drive the flow through the membrane.

During filtration, filtrate volumes of 40 mL were collected and after filtration the retentates (approximately 50 ml) were collected, freeze-dried and mass determined to estimate mass balance.

Subsequently, the original CM-Dextran and the refined CM-Dextrans from both the retentate and filtrate were analyzed for size using the method as outlined in Example 1.

Examples of the CM-Dextrans with different Mw Cut-off values are listed in the table below:

**Table 3: Carboxymethyl dextrans filtered with three different membranes and the resulting average molecular weight and average molecular weight of the lowest and highest 10% fraction.**

| ID | Sample, in retenate | Membrane cut off size, KDa | Recovery in retentate, % | Average Mol. weight, kDa | Average Mol. weight, lowest 10% fraction, kDa | Average Mol. weight, highest 10% fraction, kDa |
|---|---|---|---|---|---|---|
| 5 | CM-Dextran | None | Nd | 1958 | 819 | 5227 |
| 5a | CM-Dextran | 100 | 97 | 2139 | 900 | 5870 |
| 5b | CM-Dextran | 300 | 58 | 2634 | 1227 | 5968 |
| 5c | CM-Dextran | 500 | 54 | 2687 | 1275 | 5894 |

The molecular weight of the refined CM-Dextran may not be directly compared to the unmodified dextran as the measured size is apparently larger. For these specific membranes, no large difference between 300 and 500 kDa membranes are observed for refining the CM-Dextrans.

### Example 8

### Coupling of biotin-C17 linker to a high molecular weight CM-Dextran

The carboxymethyl dextrans (5, 5a, 5b, 5c from example 7 above, 320 µmol COOH/g, in 25 mg/ml) were activated and coupled with a biotin linker in a "one-pot" conjugation reaction using carbodiimide (16 mol eq. per mol COOH, EDAC, Merck) and N-Hydroxy succinimide (16 mol eq. per mol COOH, Aldrich) in 0.5 M MES buffer at pH 6.0 (Sigma-Aldrich) in the presence of the Biotin-C17-NH₂ linker (8 mol eq. per mol COOH, 5-((3aS, 4S, 6aR)-2-oxo-hexahydro-1H-thieno(3,4-d)imidazole-4-yl)-N-(2-(2-(3-(3-aminopropanamido)-2-methyl-propanamido)propanamido)-2-methylpropanamido)ethyl)pentanamide, hydrochloride, 635.2 g/mol, C₂₆H₄₆N₈O₆S.HCl, +98% pure according to HPLC area, obtained from NCK A/S, Denmark). The reaction mixture was mixed overnight using a tilting laboratory mixer at room temperature.

The solution was precipitated by dropwise addition to a magnetically stirred cold methanol solution (5 x volumes, 50 ml), and the biotin-dextran was collected by filtering in a Buchner funnel and washed with cold methanol. The wash was repeated 3 times. The biotin-dextran was collected and dried in a vacuum desiccator to constant weight.

A sample of the biotin-dextran, the soluble matrix, was dissolved in type 1 water and measured by UV at 205 nm. The biotin-linker content in the prepared biotin-dextrans was calculated by interpolation on a Biotin-C17-NH₂ linker standard curve.

Examples of soluble matrices of biotin-dextrans are summarized in table 5 below in Example 9.

The PBA0263 had a 30% lower biotin loading, compared to the extra refined dextrans, likely due to chemical reaction impurities. CM-dextrans further refined had slightly higher biotin-linker loading.

By further changing the stoichiometry of coupling reagents, biotin-linker or changing the pH or concentrations, the amount of linker could be varied.

### Example 9

### Determining the binding capacity

The following example illustrates the equivalence point determination for DAP molecules in solution against a biotin-linked soluble dextran matrix, as determined by SEC-HPLC.

The recombinant DAP was prepared as explained in e.g. EP2427482. The recombinant DAP molecule consists of binding domains from Protein A fused to Streptavidin

A stock solution of DAP molecules (purified recombinant protein obtained from CHRETO Aps, Denmark) was diluted to 0.80 mg/ml and titrated against a known dilution series of the biotin-linker soluble matrix in microtiter plate wells (Dx-3, Dx-5, Dx-7, Dx-9 and Dx-11) in a citrate buffer (0.1 M Citrate, 0.03 % Tween-20, pH 3.4).

Samples of the solution was analyzed by SEC-HPLC (TSKgel 300 column, Tosoh Bioscience) and the retention time and area under the curve (AUC) at 280 nm from the free DAP and the DAP bound to the biotin-linker soluble matrix were recorded.

An example (PBA0268) of the SEC-HPLC retention time and AUC is summarized in the table below and plotted in a graph, (figure 5) showing DAP binding (%)/depletion (%) as a function of the biotin-dextran concentration.

**Table 4: Retention time and area under the curve (AUC) recorded from SEC-HPLC analysis of the reaction between DAP (0.8 mg/mL) and biotin-linker-dextran at various concentrations.**

| Sample Name | Biotin dextran Conc | DAP conc. | Retention time (Dex-DAP) | AUC (Dex-DAP) | AUC (DAP) | AUC total | Residual DAP (%) | DAP depletion (%) |
|---|---|---|---|---|---|---|---|---|
| Buffer Blank | 0,0 | 0,00 | | | | | | |
| DAP blank | 0,0 | 0,80 | | | 12.619 | | | |
| DAP blank | 0,0 | 0,80 | | | 13.308 | | | PBA0268 |
| Dx-3 | 0,031 | 0,80 | 6,040 | 13.221 | | 13221 | 0,0 | 100,0 |
| Dx-5 | 0,020 | 0,80 | 6,040 | 13.647 | | 13647 | 0,0 | 100,0 |
| Dx-7 | 0,012 | 0,80 | 6,040 | 11.949 | | 11949 | 0,0 | 100,0 |
| Dx-9 | 0,006 | 0,80 | 6,048 | 7.284 | 4.215 | 11499 | 36,7 | 63,3 |
| Dx-11 | 0,004 | 0,80 | 6,048 | 5.883 | 6.856 | 12739 | 53,8 | 46,2 |

The maximum binding capacity of the soluble matrix was determined by the smallest amount of biotin matrix sufficient to reduce the free DAP AUC to zero.

The maximum binding capacity could subsequently be calculated as the maximum amount of DAP bound per biotin dextran matrix. The biotin density and the binding capacity for a number of soluble matrixes are summarized below.

**Table 5: Summary of biotin loading, number of glucose subunits between the biotin linkers and the binding capacity for DAP for 4 different soluble matrixes of biotin-Dextran. PBA0267 was not tested for DAP binding.**

| Sample | Based on CM-Dextran | Biotin loading (µg Biotin-C17 /mg dextran) | No. of glucose units per biotin | Binding Capacity DAP/Dextran mg/mg |
|---|---|---|---|---|
| PBA0263 | 5 | 69.30 | 57 | 0,211 |
| PBA0266 | 5a | 99.81 | 39 | 0,146 |
| PBA0267 | 5b | 99.67 | 39 | nd |
| PBA0268 | 5c | 99.49 | 39 | 0,147 |

The table shows a relationship between the biotin density and the binding capacity.

### Example 10

### DAP removal on Biotin resin column

For comparative purposes, the removal of DAP from a solution was carried out using a commercially available, state-of-the-art biotin affinity resin. A DAP solution (43 mg in 50 mL 0.1 M citric buffer, pH 3.4) was provided, and the DAP molecules were removed from the solution through the use of a high-performance crosslinked biotin agarose resin (12.5 ml, product code no. 4BCL-BI-100, Agarose Bead Technologies, Spain) packed in a column (XK16, Cytiva Sweden AB).

In brief, the DAP solution was introduced into the biotin agarose bead column at varying pump speeds to ensure efficient and complete DAP removal. The entire procedure was limited to a maximum of 60 minutes to minimize both the process time and the subsequent exposure of the target IgG to the low pH buffer.

The SDS-PAGE analysis was done according to manufacturer's recommendations using a Novex^{™} Tris-Glycine SDS Sample and running Buffer and NovexTM WedgeWellTM 12% Tris-Glycine Gel (1.0mm x 12 well and SeeBlueTM Protein Ladder (Plus2 Pre-stained Standard) and XCell SureLock Mini-Cell Electrophoresis System (all from Invitrogen/ThermoFisher Scientific) with LKB Bromma 2301 Macrodrive 1 Power Supply. In the typical procedure, each well was loaded with a sample (20 µl, 2.5 µg/µl) or protein standard (10 µl) and the gel run for 40 minutes at 225 V. After Coomasie staining (Serva Blue R/acetic acid/Ethanol) overnight and subsequent destaining (Glycerol/ethanol/acetic acid) and wash with type 1 water, the gel was scanned for documentation.

The efficiency of DAP removal was assessed by SDS-PAGE, revealing no detectable DAP at a flow speed of 1 ml/min. However, at a higher flow speed (4 ml/min), a trace of DAP was observed in the flow-through, as confirmed by SDS-PAGE. Attempts to increase DAP removal at a lower pump speed exceeded the 60-minute limit.

This example of a DAP removal procedure using a biotin beads system corresponds to a practical binding capacity at approximately 3.4 mg DAP /mL biotin agarose resin, but also illustrates the slower binding reaction of the resin bead-based system.

### Example 11

### DAP removal in depth filters with soluble matrix.

In this experiment the effect of having dextrans with different molecular weight (MW) values were tested in the rapid depth filter application.

DAP was captured by three different soluble matrices using a depth filter set-up. The soluble matrices (PBA0263, PBA0266 and PBA0268) originated from the same high Mw dextran, had various Mw values (refer to example 7 and 8). Additionally, an antibody sample, representing the target biomolecule, was introduced to assess the efficiency of separating DAP and the target antibody under low pH conditions with the use of the soluble matrix in a depth filter.

Samples was prepared by mixing DAP and soluble matrix with or without antibody (Privigen Immune Globulin Intravenous (Human), 10% (w/v) IgG, CSL Behring AG) as target biomolecule.

In short, biotin-linker-dextran solutions (182µL PBA0263 with 9,1 mg, 128µL PBA0266 with 6,4 mg or 128µL PBA0268 with 6,4 mg ) and DAP solution (53 mL, 0.8 mg DAP/mL, 42.7 mg) were mixed with magnetic stirring for 120 seconds. The ratios between biotin-linker-dextran and DAP was corresponding to the equivalence points.

Filter aid (6 g Filtrox C65, Advanced Minerals, in 50 mL 0.1 M Citric buffer, pH 3.4) was added to the DAP-biotin-linker-dextran solution, and the suspension was pumped (80 mL/min) into a filter house to build an even filter cake (volume of approximately 20 mL and 1 cm thickness).

The flow through (filtrate) from the filter house was collected in 40 mL fractions, and the filter cake was washed with additional buffer (2x40 mL citric buffer, pH 3.4 at pump speed 50 mL/min.) and the filtrate was collected.

The duration of DAP removal by the soluble biotinylated dextran in the depth filter procedure was 140 sec (2min 20 sec) in total.

The filtrate was analysed by UV (280 nm, Nanodrop One, Thermo Fisher) and SDS-PAGE (Se previous procedure) for free antibody and traces of DAP.

The results are summarized in the following table:

**Table 6: Summary of the removal of DAP, using three different soluble matrix, the biotin-dextrans, with and without the presence of the target biomolecule, IgG, in a depth filter.**

| | Biotin-Dextran (mg) | DAP (mg) | Target Ab (mg) | Filter Aid | DAP in retentate (mg) | DAP in Filtrate % | Target Ab recovery (%) |
|---|---|---|---|---|---|---|---|
| PBA0263 | 9.1 | 42,7 | No | C65 | 0,60 | 1,39 | |
| PBA0266 | 6.4 | 42,7 | No | C65 | 0,69 | 1,62 | |
| PBA0268 | 6.4 | 42,7 | No | C65 | 0,69 | 1,62 | |
| | | | | | | | |
| PBA0263 | 9.1 | 42,7 | 300 | C65 | 223,8 | Traces | 74,6 |
| PBA0266 | 6.4 | 42,7 | 300 | C65 | 226,3 | 0 | 75,4 |
| PBA0268 | 6.4 | 42,7 | 300 | C65 | 225,6 | 0 | 75,2 |

Capture without added IgG gave a low UV signal, which could be due to traces of DAP. Alternatively, it could be filter aid debris.

Capture of DAP using the soluble matrix PBA0266 or PBA0268 using depth filter with C65 filter aid was complete, according to SDS-PAGE. No DAP residues were visible and the antibody could be recovered at high yield. Figure 6 illustrates the SDS-PAGE analysis of the filtrate from the experiment with the soluble matrix PBA0268.

The less refined PBA0263 soluble matrix with slightly lower biotin density resulted in nearly complete capture of DAP, as only traces of DAP was found in the filtrate, according to SDS-PAGE analysis.

This example serves to demonstrate the impact of biotin-linker loadings and the Mw values of different soluble matrices, when using a depth filter.

Also, in comparison with the DAP capture using a biotin resin column procedure (Example 10), the soluble matrix and depth filter procedure is about 25 times faster. In addition, the procedure utilizes simple mixing of solutions and filtering, making process scale-up straight forward.

### EXAMPLE 12

### DAP removal in membrane filters with soluble matrix

In this experiment the utility of capturing and removing DAP by a soluble matrix was tested using a membrane filter system.

A tangential flow filtration system with a 500K membrane and 50 cm² effective filtration area was used (Pall Minimate EVO TFF and Flow Filtration Capsules, OA500C12, Pall).

In summary, a biotin-linker-dextran solution (PBA0268, 128 µL, 6.82 mg) was mixed with DAP solution (53 mL, 0.8 mg DAP/mL, 46,4 mg) in a 0.1 M citric buffer, pH 3.4 and incubated for 120 seconds on magnetic stirring.

The DAP and biotin-linker-dextran solution was loaded to the reservoir of the system and mixed with additional 300 mL 0.1M citric buffer, pH 3.4. The system was operated at 0.5-1 bar pressure to drive the flow through the membrane.

During filtration, filtrate volumes of 40 mL filtrate fractions were collected and after the filtration process, approximately 50 ml of the retentate was collected.

The effectiveness of DAP removal was assessed by analyzing the retentate and filtrate fractions by UV at 280 nm (Nanodrop One)

No free DAP nor DAP-biotin-linker-dextran was detected in the filtrate, while all DAP or DAP-biotin-linker dextran was retained in the retentate.

### EXAMPLE 13

### DAP removal in membrane filters with soluble matrix

In this experiment the utility of capturing and removing DAP by a soluble matrix was tested using a 1000K membrane filter system. Additionally, an antibody sample, representing the target biomolecule, was introduced to assess the efficiency of separating DAP and the target antibody under low pH conditions. Samples were prepared by mixing DAP and soluble matrix with antibody (Privigen Immune Globulin Intravenous (Human), 10% (w/v) IgG, CSL Behring AG,) in a 0.1 M citric buffer at pH 3.4.

A tangential flow filtration system with a 1000K membrane and 50 cm² effective filtration area was used (Minimate EVO TFF and Flow Filtration Capsules, OA990C12, Pall).

In summary, a biotin-linker-dextran solution (PBA0268, 128 µL, 6.4 mg) was mixed with DAP solution (51 mL, 42,4 mg DAP) and 3.0 mL Privigen, 300 mg IgG) in a 0.1 M citric buffer at pH 3.4 and incubated for 120 seconds with magnetic stirring.

The DAP, IgG and biotin-linker-dextran solution was loaded to the reservoir of the system and mixed with 300 mL additional buffer (0.1M citric buffer, pH 3.4). The system was operated at 0.5-1 bar pressure to drive the flow through the membrane.

During filtration, filtrate volumes of 40 mL filtrate fractions were collected, and after the filtration process approximately 80 ml of the retentate was collected.

The effectiveness of DAP removal was assessed by analysing the retentate and filtrate fractions by SDS-PAGE analysis.

Small amounts of DAP or DAP-biotin-linker-dextran were detected in the filtrate by SDS-PAGE. (Figure 7). The cut-off of the 1000K membrane was too high for complete removal in this experiment. The recovery of the IgG in the filtrate was assessed by visual inspection to be approximately 80-90% and the removal of the DAP to be better than 50%. The retentate showed a high content of DAP-biotin dextran and some IgG residue.

The example illustrates the importance of the combination of soluble matrix, DAP, target and filtration membranes. E.g. various cut-off values of the membranes can be applied to design a process that both allows a high recovery of target molecules in the flow-through and the desired level of DAP removal, as the DAP-biotin-dextran is isolated in the retentate.

### LIST OF REFERENCES

EP 2220107 B1 (CHRETO)
EP 2427482 B1 (CHRETO)
WO 2020/127311 A1
US2008108053 A1 (AFFISINK BIOTECHNOLOGY LTD), 2008-05-08
WO2020037100 A1 (ISOLERE BIO INC.), 2020-02-20
WO2012055854 A1 (SPIBER TECHNOLOGIES AB et al.), 2012-05-03
US2009232737 A1 (MOYA WILSON et al.), 2009-09-17
WO2018178991 A1 (YISSUM RES DEV CO OF HEBREW UNIV JERUSALEM LTD), 2018-10-04
WO2021168270 A1 (ISOLERE BIO INC et al.), 2021-08-26
WO2006110292 A2 (UNIV CALIFORNIA et al.), 2006-10-19
WO2009078018 A2 (AFFISINK BIOTECHNOLOGY LTD et al.), 2009-06-25
WO2008067591 A1 (INNOVATION PURIFICATION TECHNO et al.), 2008-06-12
WO2023012321 A1 (ROCHE DIAGNOSTICS GMBH et al.), 2023-02-09
WO2016049761 A1 (BIOASTRA TECHNOLOGIES INC) 2016-04-07
Nygren, H. and Stenberg, M. (1989) Immunochemistry at Interfaces. Immunology, 66, 321-327)
FORDE, G.M. (2008): "Plasmid DNA Purification Via the Use of a Dual Affinity Protein", in Zachariou, M. (eds) Affinity Chromatography, Methods in Molecular Biology, vol 421, pp. 275-284, Humana Press
MONDAL, K. et al. (2006): "The affinity concept in bioseparation: Evolving paradigms and expanding range of application", Biomolecular Engineering, Vol. 23, Issues 2-3, pp. 59-76, https://doi.org/10.1016/j.bioeng.2006.01.004

## Claims

1. A method for purification of a target biomolecule, comprising the steps:
a. Contacting i) a starting composition comprising the target biomolecule and ii) a dual affinity polypeptide (DAP), said DAP having a first binding functionality towards the target biomolecule and a second binding functionality towards a catching ligand, in solution, to allow the DAP to form complexes with the target biomolecule, wherein the ratio between the equilibrium dissociation constants of the dual affinity polypeptide, [K_{D,t}/K_{D,s}], is at least 10¹ at standard conditions;
b. Washing the formed target biomolecule-DAP complexes to remove any impurities and to form pre-purified target biomolecule-DAP complexes;
c. Treating the pre-purified target biomolecule-DAP complexes at a pH below 5.0 to separate the target biomolecule from the DAP by dissolving the 3D-complex and thereby providing a mixture of pure target biomolecules and DAP;
d. Contacting the mixture of c. with a soluble matrix having the catching ligand bound to it to form separate pure target biomolecules and separate DAP-catching ligand-matrix products; and
e. Recovering the pure target biomolecule.

2. The method according to claim 1, wherein the ratio between the equilibrium dissociation constants of the dual affinity polypeptide, [K_{D,t}/K_{D,s}], is at least 10¹ at standard conditions.

3. The method according to any one of the preceding claims, wherein the washing in step b. is performed by filtration, such as via depth filtration.

4. The method according to any one of the preceding claims, wherein the pH in step c. is in the range 2.8-4.7, preferably in the pH range 3.1-4.5, more preferably in the pH range of about 3.4-4.3.

5. The method according to any one of the preceding claims, wherein the soluble matrix is a water-soluble matrix or is soluble in an aqueous solution.

6. The method according to claim 5, wherein the soluble matrix is a polymer selected from the group consisting of dextrans, xanthan gum, pectins, chitin and chitosan, carrageenans, guar gum, cellulose ethers, hyaluronic acid, albumin, hydroxy ethyl starch and other starch derivatives, polyacrylic acid, polyacrylamides, polyvinyl alcohols, polyethylene glycols, polyvinyl pyrrolidone, divinyl ether-maleic anhydride, polyoxazoline, polyphosphates, polyphosphazenes, copolymers thereof, and mixtures thereof, preferably selected from the group consisting of dextrans, carrageenan, pectins, cellulose ethers, polyacrylic acid, polyacrylamides, polyvinyl alcohols, copolymers thereof, and mixtures thereof, more referably the soluble matrix is a dextran polymer.

7. The method according to any one of the preceding claims, wherein the difference in size between the soluble matrix and that of the target biomolecule is at least 3 times, such as a difference in size of at least 5 times, such as at least 7 times, such as at least 10 times.

8. The method according to any one of the preceding claims, wherein the difference in size between the DAP-soluble matrix and that of the target biomolecule is at least 3 times, such as a difference in size of at least 5 times, such as at least 7 times, such as at least 10 times.

9. The method according to any one of the preceding claims, wherein the soluble matrix is connected to the catching ligand via a linker having a linker length of 10-25 bonds, such as a length of 10-24 bonds, such as a length of 10-23 bonds, such as a length of 10-22 bonds, such as a length of 10-21 bonds, such as a length of 10-20 bonds, such as a length of 10-19 bonds, such as a length of 10-18 bonds, such as a length of 10-17 bonds, such as a length of 10-16 bonds, such as a length of 10-15 bonds, such as a length of 10-14 bonds, such as a length of 10-13 bonds, such as a length of 10-12 bonds, such as a length of 10-11 bonds, such as a length of 11-25 bonds, such as a length of 12-25 bonds, such as a length of 13-25 bonds, such as a length of 14-25 bonds, such as a length of 15-25 bonds, such as a length of 16-25 bonds, such as a length of 17-25 bonds, such as a length of 18-25 bonds, such as a length of 19-25 bonds, such as a length of 20-25 bonds, such as a length of 21-25 bonds, such as a length of 22-25 bonds, such as a length of 23-25 bonds, such as a length of 24-25 bonds.

10. The method according to claim 9, wherein the linker is selected from the group consisting of 2-Vinyl-4,4-dimethyl-5-oxazolone (VDMA), vinyl azlactone derivatives, acrylic derivatives, peptides, polyamides, hexandiisocyanate (HDI) derivatives, diisocyanate derivatives, or mixtures hereof.

11. The method according to any one of the preceding claims, wherein the catching ligand is selected from the group consisting of biotin, and biotin analogues.

12. The method according to any one of the preceding claims, wherein step e. is performed using filtration, such as membrane filtration, diafiltration, cross-flow diafiltration, hollow fibre filtration, depth filtration, microfiltration or ultrafiltration.

13. The method according to any one of the preceding claims, wherein the target biomolecule is a protein, preferably an antibody, a virus particle or exosomes, cells or cell components.

14. The method according to any one of the preceding claims, wherein at least steps c.-e. of the method, and preferably the entire method, is/are performed as a continuous operation.

## Patentansprüche

1. Verfahren zur Reinigung eines Zielbiomoleküls, umfassend die Schritte:
a. Inkontaktbringen von i) einer Ausgangszusammensetzung, die das Zielbiomolekül umfasst, und ii) einem Polypeptid mit dualer Affinität (DAP), wobei das DAP eine erste Bindungsfunktionalität gegenüber dem Zielbiomolekül und eine zweite Bindungsfunktionalität gegenüber einem Fangliganden aufweist, in Lösung, um dem DAP zu ermöglichen, Komplexe mit dem Zielbiomolekül zu bilden, wobei das Verhältnis zwischen den Gleichgewichts-Dissoziationskonstanten des Polypeptids mit dualer Affinität, [K_{D,t}/K_{D,s}], unter Standardbedingungen mindestens 10¹ beträgt;
b. Waschen der gebildeten Zielbiomolekül-DAP-Komplexe, um etwaige Verunreinigungen zu entfernen und vorreinigte Zielbiomolekül-DAP-Komplexe zu bilden;
c. Behandeln der vorreinigten Zielbiomolekül-DAP-Komplexe bei einem pH-Wert unter 5,0, um das Zielbiomolekül von dem DAP zu trennen, indem der 3D-Komplex aufgelöst wird und dadurch eine Mischung aus reinen Zielbiomolekülen und DAP bereitgestellt wird;
d. Inkontaktbringen der Mischung aus c. mit einer löslichen Matrix, an die der Fangligand gebunden ist, um separate reine Zielbiomoleküle und separate DAP-Fangligand-Matrix-Produkte zu bilden; und
e. Gewinnen des reinen Zielbiomoleküls.

2. Verfahren nach Anspruch 1, wobei das Verhältnis zwischen den Gleichgewichts-Dissoziationskonstanten des Polypeptids mit dualer Affinität, [K_{D,t}/K_{D,s}], unter Standardbedingungen mindestens 10¹ beträgt.

3. Verfahren nach einem der vorhergehenden Ansprüche, wobei das Waschen in Schritt b. durch Filtration, wie zum Beispiel über Tiefenfiltration, durchgeführt wird.

4. Verfahren nach einem der vorhergehenden Ansprüche, wobei der pH-Wert in Schritt c. im Bereich von 2,8 - 4,7, bevorzugt im pH-Bereich von 3,1 - 4,5 und mehr bevorzugt im pH-Bereich von ungefähr 3,4 - 4,3 liegt.

5. Verfahren nach einem der vorhergehenden Ansprüche, wobei die lösliche Matrix eine wasserlösliche Matrix ist oder in einer wässrigen Lösung löslich ist.

6. Verfahren nach Anspruch 5, wobei die lösliche Matrix ein Polymer ist, das aus der Gruppe ausgewählt ist, die aus Dextranen, Xanthangummi, Pektinen, Chitin und Chitosan, Carrageenanen, Guargummi, Celluloseethern, Hyaluronsäure, Albumin, Hydroxyethylstärke und anderen Stärkederivaten, Polyacrylsäure, Polyacrylamiden, Polyvinylalkoholen, Polyethylenglykolen, Polyvinylpyrrolidon, Divinylether-Maleinsäureanhydrid, Polyoxazolin, Polyphosphaten, Polyphosphazenen, Copolymeren davon und Mischungen davon besteht, bevorzugt aus der Gruppe ausgewählt ist, die aus Dextranen, Carrageen, Pektinen, Celluloseethern, Polyacrylsäure, Polyacrylamiden, Polyvinylalkoholen, Copolymeren davon und Mischungen davon besteht, wobei die lösliche Matrix mehr bevorzugt ein Dextranpolymer ist.

7. Verfahren nach einem der vorhergehenden Ansprüche, wobei der Größenunterschied zwischen der löslichen Matrix und dem Zielbiomolekül mindestens das Dreifache beträgt, wie zum Beispiel ein Größenunterschied von mindestens dem Fünffachen, wie zum Beispiel mindestens dem Siebenfachen, wie zum Beispiel mindestens dem Zehnfachen.

8. Verfahren nach einem der vorhergehenden Ansprüche, wobei der Größenunterschied zwischen der DAP-löslichen Matrix und dem Zielbiomolekül mindestens das Dreifache beträgt, wie zum Beispiel ein Größenunterschied von mindestens dem Fünffachen, wie zum Beispiel mindestens dem Siebenfachen, wie zum Beispiel mindestens dem Zehnfachen.

9. Verfahren nach einem der vorhergehenden Ansprüche, wobei die lösliche Matrix mit dem Fangliganden über einen Linker mit einer Linkerlänge von 10-25 Bindungen verbunden ist, wie zum Beispiel mit einer Länge von 10-24 Bindungen, wie zum Beispiel mit einer Länge von 10-23 Bindungen, wie zum Beispiel einer Länge von 10-22 Bindungen, wie zum Beispiel einer Länge von 10-21 Bindungen, wie zum Beispiel einer Länge von 10-20 Bindungen, wie zum Beispiel einer Länge von 10-19 Bindungen, wie zum Beispiel einer Länge von 10-18 Bindungen, wie zum Beispiel einer Länge von 10-17 Bindungen, wie zum Beispiel einer Länge von 10-16 Bindungen, wie zum Beispiel einer Länge von 10-15 Bindungen, wie zum Beispiel einer Länge von 10-14 Bindungen, wie zum Beispiel einer Länge von 10-13 Bindungen, wie zum Beispiel einer Länge von 10-12 Bindungen, wie zum Beispiel einer Länge von 10-11 Bindungen, wie zum Beispiel einer Länge von 11-25 Bindungen, wie zum Beispiel einer Länge von 12-25 Bindungen, wie zum Beispiel einer Länge von 13-25 Bindungen, wie zum Beispiel einer Länge von 14-25 Bindungen, wie zum Beispiel einer Länge von 15-25 Bindungen, wie zum Beispiel einer Länge von 16-25 Bindungen, wie zum Beispiel einer Länge von 17-25 Bindungen, wie zum Beispiel einer Länge von 18-25 Bindungen, wie zum Beispiel einer Länge von 19-25 Bindungen, wie zum Beispiel einer Länge von 20-25 Bindungen, wie zum Beispiel einer Länge von 21-25 Bindungen, wie zum Beispiel einer Länge von 22-25 Bindungen, wie zum Beispiel einer Länge von 23-25 Bindungen, wie zum Beispiel einer Länge von 24-25 Bindungen.

10. Verfahren nach Anspruch 9, wobei der Linker aus der Gruppe ausgewählt ist, die aus 2-Vinyl-4,4-dimethyl-5-oxazolon (VDMA), Vinyl-Azlacton-Derivaten, Acrylderivaten, Peptiden, Polyamiden, Hexandiisocyanat (HDI)-Derivaten, Diisocyanat-Derivaten oder Mischungen davon besteht.

11. Verfahren nach einem der vorhergehenden Ansprüche, wobei der Fangligand aus der Gruppe ausgewählt ist, die aus Biotin und Biotinanaloga besteht.

12. Verfahren nach einem der vorhergehenden Ansprüche, wobei Schritt e. unter Verwendung von Filtration durchgeführt wird, wie zum Beispiel Membranfiltration, Diafiltration, Querstrom-Diafiltration, Hohlfaserfiltration, Tiefenfiltration, Mikrofiltration oder Ultrafiltration.

13. Verfahren nach einem der vorhergehenden Ansprüche, wobei das Zielbiomolekül ein Protein, bevorzugt ein Antikörper, ein Viruspartikel oder Exosomen, Zellen oder Zellbestandteile ist.

14. Verfahren nach einem der vorhergehenden Ansprüche, wobei mindestens die Schritte c. - e. des Verfahrens und bevorzugt das gesamte Verfahren als kontinuierlicher Vorgang durchgeführt werden.

## Revendications

1. Procédé de purification d'une biomolécule cible, comprenant les étapes suivantes :
a. Mettre en contact i) une composition de départ comprenant la biomolécule cible et ii) un polypeptide à double affinité (DAP), ledit DAP présentant une première fonctionnalité de liaison vis-à-vis de la biomolécule cible et une seconde fonctionnalité de liaison vis-à-vis d'un ligand de capture, en solution, afin de permettre au DAP de former des complexes avec la biomolécule cible, dans lequel le rapport entre les constantes de dissociation à l'équilibre du polypeptide à double affinité, [K_{D,t}/K_{D,s}], est d'au moins 10¹ dans des conditions standard ;
b. Laver les complexes biomolécule cible-DAP formés afin d'éliminer toute impureté et de former des complexes biomolécule cible-DAP pré-purifiés ;
c. Traiter les complexes biomolécule cible-DAP pré-purifiés à un pH inférieur à 5,0 afin de séparer la biomolécule cible du DAP en dissolvant le complexe 3D et ainsi obtenir un mélange de biomolécules cibles pures et de DAP ;
d. Mettre en contact le mélange de l'étape c. avec une matrice soluble comportant le ligand de capture qui lui est lié, afin de former des biomolécules cibles pures distinctes et des produits distincts DAP-ligand de capture-matrice ; et
e. Récupérer la biomolécule cible pure.

2. Procédé selon la revendication 1, dans lequel le rapport entre les constantes de dissociation à l'équilibre du polypeptide à double affinité, [K_{D,t}/K_{D,s}], est d'au moins 10¹ dans des conditions standard.

3. Procédé selon l'une des revendications précédentes, dans lequel le lavage de l'étape b. est effectué par filtration, par exemple par filtration en profondeur.

4. Procédé selon l'une des revendications précédentes, dans lequel le pH à l'étape c. se situe dans la plage de 2,8 à 4,7, de préférence dans la plage de pH de 3,1 à 4,5, et plus préférentiellement dans la plage de pH d'environ 3,4 à 4,3.

5. Procédé selon l'une des revendications précédentes, dans lequel la matrice soluble est une matrice soluble dans l'eau ou est soluble dans une solution aqueuse.

6. Procédé selon la revendication 5, dans lequel la matrice soluble est un polymère choisi dans le groupe constitué par les dextranes, la gomme xanthane, les pectines, la chitine et le chitosane, les carraghénanes, la gomme de guar, les éthers de cellulose, l'acide hyaluronique, l'albumine, l'amidon hydroxyéthylé et d'autres dérivés de l'amidon, l'acide polyacrylique, les polyacrylamides, les alcools polyvinyliques, les polyéthylène-glycols, la polyvinylpyrrolidone, l'éther divinylique-anhydride maléique, la polyoxazoline, les polyphosphates, les polyphosphazènes, leurs copolymères et leurs mélanges, de préférence choisi dans le groupe constitué par les dextranes, le carraghénane, les pectines, les éthers de cellulose, l'acide polyacrylique, les polyacrylamides, les alcools polyvinyliques, leurs copolymères et leurs mélanges, plus préférentiellement, la matrice soluble est un polymère de dextrane.

7. Procédé selon l'une des revendications précédentes, dans lequel la différence de taille entre la matrice soluble et celle de la biomolécule cible est d'au moins 3 fois, par exemple une différence de taille d'au moins 5 fois, par exemple d'au moins 7 fois, par exemple d'au moins 10 fois.

8. Procédé selon l'une des revendications précédentes, dans lequel la différence de taille entre la matrice soluble-DAP et celle de la biomolécule cible est d'au moins 3 fois, par exemple une différence de taille d'au moins 5 fois, par exemple d'au moins 7 fois, par exemple d'au moins 10 fois.

9. Procédé selon l'une des revendications précédentes, dans lequel la matrice soluble est reliée au ligand de capture par l'intermédiaire d'un lieur présentant une longueur de 10 à 25 liaisons, par exemple une longueur de 10 à 24 liaisons, par exemple une longueur de 10 à 23 liaisons, par exemple une longueur de 10 à 22 liaisons, par exemple une longueur de 10 à 21 liaisons, par exemple une longueur de 10 à 20 liaisons, par exemple une longueur de 10 à 19 liaisons, par exemple une longueur de 10 à 18 liaisons, par exemple une longueur de 10 à 17 liaisons, par exemple une longueur de 10 à 16 liaisons, par exemple une longueur de 10 à 15 liaisons, par exemple une longueur de 10 à 14 liaisons, par exemple une longueur de 10 à 13 liaisons, par exemple une longueur de 10 à 12 liaisons, par exemple une longueur de 10 à 11 liaisons, par exemple une longueur de 11 à 25 liaisons, par exemple une longueur de 12 à 25 liaisons, par exemple une longueur de 13 à 25 liaisons, par exemple une longueur de 14 à 25 liaisons, par exemple une longueur de 15 à 25 liaisons, par exemple une longueur de 16 à 25 liaisons, par exemple une longueur de 17 à 25 liaisons, par exemple une longueur de 18 à 25 liaisons, par exemple une longueur de 19 à 25 liaisons, par exemple une longueur de 20 à 25 liaisons, par exemple une longueur de 21 à 25 liaisons, par exemple une longueur de 22 à 25 liaisons, par exemple une longueur de 23 à 25 liaisons, par exemple une longueur de 24 à 25 liaisons.

10. Procédé selon la revendication 9, dans lequel le lieur est choisi dans le groupe constitué par la 2-vinyl-4,4-diméthyl-5-oxazolone (VDMA), des dérivés de vinylazlactone, des dérivés acryliques, des peptides, des polyamides, des dérivés d'hexanediisocyanate (HDI), des dérivés de diisocyanate, ou des mélanges de ceux-ci.

11. Procédé selon l'une des revendications précédentes, dans lequel le ligand de capture est choisi dans le groupe constitué par la biotine et des analogues de la biotine.

12. Procédé selon l'une des revendications précédentes, dans lequel l'étape e. est effectuée par filtration, par exemple par filtration sur membrane, diafiltration, diafiltration à flux croisé, filtration sur fibres creuses, filtration en profondeur, microfiltration ou ultrafiltration.

13. Procédé selon l'une des revendications précédentes, dans lequel la biomolécule cible est une protéine, de préférence un anticorps, une particule virale ou des exosomes, des cellules ou des composants cellulaires.

14. Procédé selon l'une des revendications précédentes, dans lequel au moins les étapes c) à e) du procédé, et de préférence l'ensemble du procédé, sont réalisées en continu.
